# EUROPEAN PATENT SPECIFICATION

(11) **EP 1 255 518 B1**
(45) Date of publication and mention of the grant of the patent: **24.08.2011**
(21) Application number: 01904761.2
(22) Date of filing: 14.02.2001
(51) Int. Cl.: A61F 5/00

(54) **HEARTBURN AND REFLUX DISEASE TREATMENT APPARATUS**
VORRICHTUNG ZUM BEHANDELN VON SODBRENNEN UND REFLUXERKRANGUNGEN
APPAREIL POUR LE TRAITEMENT DU PYROSIS ET DE LA MALADIE DU REFLUX

(30) Priority: 14.02.2000 US 504047
(43) Date of publication of application: 13.11.2002
(73) Proprietor: Obtech Medical AG, 6341 Baar (CH)
(72) Inventor: FORSELL, Peter, CH-6313 Menzingen (CH)
(74) Representative: Mercer, Christopher Paul
(86) International application number: PCT/SE2001/000311
(87) International publication number: WO 2001/047435

(56) References cited:
- WO-A-01/49245
- WO-A1-00/09049
- WO-A1-00/15158
- WO-A1-01/12078
- US-A- 4 271 827
- US-A- 5 771 903
- US-A- 5 938 669

## Description

The present invention relates to a heartburn and reflux disease treatment apparatus, comprising an adjustable restriction device adapted to engage the stomach close to the cardia or esophagus of a patient to form a restricted food passageway in the stomach or esophagus. The term "patient" includes an animal or a human being.

Chronic heartburn and reflux disease is a widespread medical problem. This is often due to hiatal hernia, i.e. a portion of the stomach immediately below the gastric fundus slides upwardly through the esophageal hiatus. In consequence, stomach acids and foods are regurgitated into the esophagus.

In the late 1970s a prior art prosthesis called Angelchik, according to U.S. Patent No. 3875928, was used to operatively treat heartburn and reflux disease. However, the Angelchik prosthesis had a major disadvantage in that it was not possible to adjust the size of the restriction opening after the operation. A further disadvantage was that the prosthesis did not satisfactorily protect the esophagus and the surrounding area against injuries due to poor shape of the prosthesis. Moreover, the prosthesis was sutured to the stomach, in order to be properly positioned. Such a suture arrangement, however, is not reliable. Therefore, operations using the Angelchik prosthesis are no longer practised.

An operation technique, semi-fundoduplicatio, is currently in use for treating heartburn and reflux disease. A most common operation is Nissen semi-fundoduplicatio, in which one takes the fundus of the stomach and makes a three quarter of a turn around the esophagus and suture between the stomach and esophagus. Although this operation works fairly well it has three main disadvantages. Firstly, most patients treated in accordance to "ad modum Nissen" lose their ability to belch. Secondly, many of these patients get dysphagia, i.e. have difficulties in swallowing after the operation. Thirdly, it is not possible to adjust the food passageway in the esophagus or stomach in any way after the operation. Characteristic for these patients is the variation of their problems over the course of a day. For example, many patients have difficulties during the night when they lie down because of stomach acid leaking up into the esophagus.

Document WO01/49245, belonging to the state of the art in accordance with Art. 54(3)EPC, represents the closest prior art. It describes a heart burn and reflux disease treatment device similar to the device defined in claim 1 of the present invention, but the operation of the device occurs in response to pressure changes external to the implantable device.

Documents US 5938669 and US 5771903 describe treatment apparatuses comprising implantable adjustable restriction devices to engage the stomach of a patient. Similar devices are also disclosed in WO 01/12078 and WO 00/09049.

The object of the present invention is to provide a new heartburn and reflux disease treatment apparatus, which eliminates the above noted problems of the known technique for treating heartburn and reflux disease.

This object is obtained by a heartburn and reflux disease treatment apparatus of the kind stated initially characterised by an implantable adjustment device for post-operation adjustment of the restriction device to enlarge and restrict the food passageway, when the restriction device is implanted in the patient, and an implantable hydraulic operation means for operating the adjustment device. As a result, the restriction device can be individually adjusted by the adjustment device one or a few times after the operation by operating the hydraulic operation means, so that a suitable restriction of the food passageway is obtained for every patient. Thus, the final restriction of the food passageway calibrated in this manner will reduce or completely eliminate the risk of stomach acids or foods regurgitating into the esophagus while the patient still is able to eat. Of course, in accordance with prior art the restriction device may suitably be provided with an inner cushion member, which is deformable to permit normal enlargement of the food passageway during swallowing.

As an alternative to the procedure of providing one or a few adjustments of the restriction device immediately after the operation, the restriction device may advantageously be adjusted by the adjustment device to enlarge the food passageway when the patient eats and to restrict or close the food passageway between meals. In accordance with this alternative the restriction device performs like an artificial sphincter, which may be activated by the patient in connection with every food intake during the day, or possibly only in the morning to open up the food passageway and in the evening to close the food passageway.

Preferably, however the restriction device is activated by any automatic means, for instance including a sensor for sensing a physical parameter of the patient, such as the pressure in the food passageway. Suitably the restriction device is powered and controlled in a non-manual manner. The expression powered should be understood as energised with everything without manual force, preferably electric energy. The expression "non-manual manner" should be understood to mean that the restriction device is not adjusted by manually touching subcutaneously implanted components of the device, nor manipulated by touching the skin of the patient.

Preferably, the adjustment device adjusts the restriction device in a non-invasive manner.

The adjustment device may adjust the restriction device in a non-magnetic manner, i.e. magnetic forces may not be involved when adjusting the restriction device.

The adjustment device may also adjust the restriction device in a non-thermal manner, i.e. thermal energy may not be involved when adjusting the restriction device.

Generally the implanted restriction device comprises a holding device for preventing the region of the cardia to pass through the esophagal hiatus diaphragmatica. This could be achieved by an enlarged area of the esophagus and/or the restriction device that prevents the esophagus from passing the hole in the diaphragmatic muscle where the esophagus passes (a triangular opening surrounded by the crus muscles) or by fixing or holding the region of the cardia in place. The holding device may take the shape of a support member that provides a support for the restriction device upwardly against the diaphragm muscle. Alternatively, the holding device may comprise sutures, or the restriction device itself could be shaped to prevent the region of the cardia from sliding up. It would also be possible to provide means for narrowing the triangular opening. The prosthesis may comprise at least one holding device implantable in the patient for holding the esophagus or stomach in a position where the left and right crus muscles are located, to prevent the region of the cardia from moving cranial through the diaphragm muscle. The holding device is normally placed in engagement with the food restriction apparatus.

Preferably, the operation device comprises a powered operation device.

Alternatively, or in combination with a powered operation device, the servo means may be used, which enables manual manipulation without need for strong manipulation forces. The servo means may comprise hydraulic means, electric control means, magnetic means, or mechanical means, which may be activated by manual manipulating means. Using a servo system will save the use of force when adjusting the adjustment device, which may be of importance in many applications.

The term "servo means" encompasses the normal definition of a servo mechanism, i.e. an automatic device that controls large amounts of power by means of very small amounts of power, but may alternatively or additionally encompass the definition of a mechanism that transfers a weak force acting on a moving element having a long stroke into a strong force acting on another moving element having a short stroke. The servo means may comprise a motor, preferably an electric motor, which may be reversible.

In accordance with the invention, the apparatus comprises a reservoir, containing a predetermined amount of hydraulic fluid, also implantable in the patient, wherein the operation device, suitably electrically powered, operates the adjustment device by using the hydraulic fluid of the reservoir.

The adjustment device comprises an expandable cavity in the restriction device, wherein the stomach or esophagus is sqeezed upon expansion of the cavity and released upon contraction of the cavity. The operation device is adapted to distribute hydraulic fluid from the reservoir to expand the cavity, and from the cavity to the reservoir to contract the cavity.

A fluid distribution tube may readily be connected between the reservoir and the cavity in a manner so that the tube does not interfere with other implanted components of the apparatus.

The reservoir defines a chamber for the predetermined amount of fluid and the operation device changes the volume of the chamber. The operation device suitably comprises first and second wall portions of the reservoir and is adapted to provide relative displacement between the first and second wall portions of the reservoir, in order to change the volume of the chamber.

The operation device is adapted to provide said relative displacement in response to the pressure in the reservoir. Suitably, the operation device comprises a pressure controlled hydraulic operation device. For safety, an alarm may be provided for generating an alarm signal in response to the lapse of a predetermined time period during which the pressure controlling the hydraulic operation device exceeds a predetermined high value.

Suitably, the operation device is adapted to distribute fluid from the reservoir to the cavity of the restriction member in response to a predetermined first displacement of the first wall portion of the reservoir relative to the second wall portion of the reservoir and may distribute fluid from the cavity to the reservoir in response to a predetermined second displacement of the first wall portion relative to the second wall portion. The first and second wall portions (66) of the reservoir may also be designed to be displaceable relative to each other by manual manipulation thereof.

The first and second wall portions of the reservoir may be displaceable relative to each other by a magnetic, hydraulic, or electric power means, such as an electric motor. according to the invention no pump is used, only the volume of the reservoir is varied. This is of great advantage compared to the solution described below when the operation device comprises a pump used to pump fluid between the reservoir and the adjustment device because there is no need for a non-return valve and it is still possible to have fluid going both to and from the reservoir. Thus, the significant risk of malfunction when using such a non-return valve implanted in the patient is eliminated.

The operation device may comprise hydraulic means and a fluid conduit extending between the hydraulic means and the adjustment device. The hydraulic means and conduit are devoid of any non-return valve. The reservoir may form part of the conduit and a fluid chamber with a variable volume. The operation device may distribute fluid from the fluid chamber to the adjustment device by reduction of the volume of the chamber and withdraw fluid from the adjustment device by expansion of the volume of the chamber. The operation device preferably comprises a motor for moving a movable wall of the reservoir for changing the volume of the chamber. Any kind of motor could be used for the different operations as well as wireless remote solutions for controlling the operations.

The restriction device preferably is operable to perform a reversible function and accordingly there is a reversing device implantable in the patient for reversing the function performed by the restriction device. Such a reversing function preferably involves enlarging and restricting the food intake passageway by the restriction device, suitably in a stepless manner. In this connection, the control device suitably controls the reversing device, which may include a switch, to reverse the function performed by the restriction device. The reversing device may comprise hydraulic means including a valve for shifting the flow direction of a fluid in the hydraulic means. Alternatively, the reversing device may comprise a mechanical reversing device, such as a switch or a gearbox.

Where the reversing device comprises a switch the control device suitably controls the operation of the switch by shifting polarity of released energy supplied to the switch. The switch may comprise an electric switch and the source of energy may supply electric energy for the operation of the switch. The switch mentioned above may comprise an electronic switch or, where applicable, a mechanical switch.

Where the operation device comprises a motor, the reversing device is adapted to reverse the motor.

In an example not according to the invention, the operation device comprises a pump for pumping fluid between the reservoir and the adjustment device. A mechanical solution is proposed in which it is possible to pump fluid from the reservoir to the adjustment device and vice versa just by pushing an activation member in one direction. The pump preferably comprises a first activation member for activating the pump to pump fluid from the reservoir to the adjustment device, and a second activation member for activating the pump to pump fluid from the adjustment device to the reservoir. At least one of the first and second activation members may be operable by manual manipulation, preferably to permit manual pushing, pulling or rotation thereof in one direction, or by a device powered magnetically, hydraulically, or electrically (e.g. by an electric motor), or be operable by a combination of these methods. Suitably, at least one of the activation members may be adapted to operate when subjected to an external pressure exceeding a predtermined magnitude.

Another alternative is a pump pumping in only one direction and an adjustable valve to change the direction of fluid to either increase or decrease the amount of fluid in the reservoir. This valve may be manipulated either manually, mechanically, magnetically, or hydraulically.

The main embodiment of the invention described above including the reservoir may alternatively be equipped with a servo means comprising a reverse servo. The term "reverse servo" is to be understood as a mechanism that transfers a strong force acting on a moving element having a short stroke into a weak force acting on another moving element having a long stroke; i.e. the reverse function of the above-defined alternative mechanism of a normal servo mechanism. A first closed hydraulic system that controls another closed hydraulic system in which hydraulic means of the adjustment device is incorporated may be used. Minor changes in the amount of fluid in a smaller reservoir of the first system could then be transferred by the reverse servo into major changes in the amount of fluid in a larger reservoir in the second system. In consequence, the change of volume in the larger reservoir of the second system affects the hydraulic means of the adjustment device. For example, a short stroke that decreases the volume of the smaller reservoir will cause the larger reservoir to supply the adjustment device with a large amount of hydraulic fluid, which in turn results in a long mechanical adjustment stroke on the restriction device.

The great advantage of using such a reverse servo is that the larger volume system could be placed inside the abdomen or retroperitoneum where there is more space and still it would be possible to use manual manipulation means of the smaller system subcutaneously. The smaller reservoir could be controlled directly or indirectly by a fluid supply means. The fluid supply means may include another small reservoir, which may be placed subcutaneously and may be activated by manual manipulation means. Both the normal servo means and the specific reverse servo may be used in connection with all of the various components and solutions described in the present specification.

Thus, the reverse servo may be adapted to provide relative displacement between the first and second wall portions of the reservoir, suitably in response to the pressure in the reservoir, in order to change the volume of the chamber of the reservoir.

Generally, the servo means, including the reverse servo, comprises a pressure controlled servo means. The alarm mentioned above may alternatively be adapted to generate an alarm signal in response to the lapse of a predetermined time period during which the pressure controlling the servo means exceeds a predetermined high value.

The reverse servo may comprise magnetic means, electric means or manual manipulation means or a combination thereof. Preferably, however, the reverse servo comprises hydraulic means.

In accordance with a particular embodiment of the invention, the reverse servo further comprises a servo reservoir defining a chamber containing servo fluid, and the operation device comprise first and second wall portions of the servo reservoir, which are displaceable relative to each other to change the volume of the chamber of the servo reservoir. The first and second wall portions of the servo reservoir may be displaceable relative to each other by magnetic means, hydraulic means, or electric control means.

Where the reverse servo comprises hydraulic means it may further comprise a fluid supply reservoir connected to the servo reservoir in a closed system and containing a further predetermined amount of fluid. The fluid supply reservoir defines a chamber for the further predetermined amount of fluid and the operation device is adapted to change the volume of the chamber and thereby control the amount of fluid in the servo reservoir. The fluid supply reservoir comprises first and second wall portions, which are displaceable relative to each other to change the volume of the chamber of the fluid supply reservoir. Suitably, the fluid supply reservoir increases the amount of fluid in the servo reservoir in response to a predetermined first displacement of the first wall portion of the fluid supply reservoir relative to the second wall portion of the fluid supply reservoir and decreases the amount of fluid in the servo reservoir in response to a predetermined second displacement of the first wall portion of the fluid supply reservoir relative to the second wall portion of the fluid supply reservoir.

In accordance with an embodiment of the invention, the adjustment device comprises a hydraulic adjustment device, and an implantable reservoir containing a predetermined amount of hydraulic fluid and a conduit providing fluid connection between the reservoir and the hydraulic adjustment device are provided. The operation device is adapted to operate the hydraulic adjustment device by distributing hydraulic fluid through the conduit between the reservoir and the hydraulic adjustment device, wherein the conduit and hydraulic adjustment device are devoid of any non-return valve to permit free flow of hydraulic fluid in both directions in the conduit. Preferably, the reservoir forms a fluid chamber with a variable volume, and the operation device is adapted to distribute fluid from the chamber to the adjustment device by reduction of the volume of the chamber and to withdraw fluid from the adjustment device by expansion of the volume of the chamber. The operation device may comprise a motor or a pump. Alternatively, the operation device may comprise a movable wall of the reservoir for changing the volume of the chamber. For example, the operation device may be adapted to change the volume of the chamber by moving the movable wall in response to the pressure in the chamber.

In the above embodiments including a reservoir for hydraulic fluid an injection port may be provided for subcutaneous implantation in the patient to be in fluid communication with the chamber of the reservoir. The injection port may be integrated in the reservoir. Such an injection port may be provided for enabling, normally single, once-and-for-all, calibration of the amount of fluid in the hydraulic system used. The adjustment means may comprise an expandable cavity in the restriction means and the food passageway may be restricted upon expansion of the cavity and enlarged upon contraction of the cavity and the hydraulic operation means may comprise an injection port implantable subcutaneously in the patient for transcutaneously adding fluid to and withdrawing fluid from the cavity. The adjustment means may comprise an hydraulic operation means and the hydraulic operation means may comprise an injection port implantable subcutaneously in the patient for transcutaneously adding fluid to and withdrawing fluid to the hydraulic operation means. The injection port may be used only for calibration purposes and the adjustment device is further able to increase the foood restriction opening when food should pas when the patient is eating and swallows food. If a servo is used the servo may be hydraulically operated and the injection port used only for calibration purposes of the servo.

In the various embodiments hereinafter described the restriction device generally forms an at least substantially closed loop. However, the restriction device may take a variety of different shapes, such as the shape of a square, rectangle or ellipse. The substantially closed loop could for example be totally flat, i.e. thin as seen in the radial direction. The shape of restriction device may also be changed during use, by rotation or movements of the restriction device in any direction. A physical lumen, like the stomach or esophagus, often is easier to restrict by contracting two opposite sidewalls of the lumen against each other. Thus, the restriction device may be designed to perform such a contracting effect of the opposite walls of the stomach or esophagus. Either mechanical or hydraulic solutions may be employed to operate the restriction device. Alternatively, the restriction device may comprise an adjustable cuff, a clamp or a roller for bending or rotating the stomach or esophagus to close its passageway. Such a cuff, clamp or roller may also be utilized for squeezing the stomach or esophagus against human material inside the body of the patient, for example the sacral bone of the patient, or against implanted structures of the apparatus. The bending or rotating members may take any shape and be either hydraulic or non-inflatable.

Preferably the restriction device comprises an elongated restriction member and forming means for forming the restriction member into at least a substantially closed loop around the stomach or esophagus, wherein the loop defines a restriction opening, whereby the adjustment device adjusts the restriction member in the loop to change the size of the restriction opening.

Advantageously, the forming means may form the restriction member into a loop having a predetermined size. Alternatively, the forming means may form the restriction member into a loop having a size selected from several predetermined sizes.

The adjustment device may change the size of the restriction opening such that the outer circumferential confinement surface of the restriction member either is changed or is unchanged.

The elongated restriction member may be flexible, for example take the shape of a belt or cord, and the adjustment device may pull a first portion of the flexible restriction member from a second portion of the flexible restriction member opposite the first portion in the loop to squeeze the stomach or esophagus between the opposite lengths of the elongated flexible restriction member to restrict the food intake passageway. The restriction member may be non-inflatable, and the adjustment device may mechanically adjust the restriction member in the loop.

The adjustment device may mechanically or hydraulically adjust the restriction device. In the embodiments described the adjustment device may either mechanically or hydraulically adjust the restriction device, where applicable. It should be noted that the operation device might mechanically or hydraulically operate the adjustment device irrespectively of whether the adjustment device is adapted to adjust the restriction device mechanically or hydraulically.

In accordance with an embodiment of the invention, the restriction device comprises at least two elements on opposite or different sides of the stomach or esophagus, and the adjustment device decreases the distance between the elements to squeeze the stomach or esophagus between the elements, thereby restricting the food intake passageway. It is also possible to use only one element and squeeze the stomach or esophagus against human bone or tissue. The elements above may as well as all the restriction members mentioned in this application be everything from rigid to soft.

In accordance with an alternative, the restriction device bends or rotates a portion of the stomach or esophagus to restrict the food intake passageway in the same. For example, the restriction device may comprise at least two bending members, such as cylindrical or hour-glass shaped rollers, positioned on opposite or different sides of the stomach or esophagus and displaced relative to each other along the stomach or esophagus, and the adjustment device may move the bending members against the stomach or esophagus to bend the latter to restrict the food intake passageway. The restriction device may also rotate a portion of the stomach or esophagus. The bending or rotating members may take any shape and be either hydraulic or non-inflatable.

Alternatively, the two bending members one placed more distal than the other may be rotated in opposite directions relative to each other. With interconnecting means for example flexible bands between the bending members a restriction will occur between the bending members when they are rotated.

Preferably the adjustment device is operable to adjust the restriction device to steplessly change the restriction of the food intake passageway in the stomach or esophagus.

All embodiments according to the invention may be controlled by a wireless remote control.

In accordance with an advantageous embodiment of the invention, there is provided a wireless remote control for non-invasively controlling the operation device. The remote control may conveniently comprise an external hand-held remote control unit, which is manually operable by the patient to control the restriction device to squeeze and release the stomach or esophagus. With the wireless remote control the apparatus of the invention is conveniently controlled by the patient when he so desires, which is of great advantage compared to the prior art procedures. With the remote control the apparatus of the invention is conveniently controlled to adjust the implanted restriction device to release the food intake passageway when the patient wants to relieve himself or herself.

The remote control may advantageously be capable of obtaining information related to important parameters, such as the condition of the food intake passageway or the pressure against the restriction device, and of commanding the operation device to operate the adjustment device to adjust the restriction device in response to obtained information. With the remote control the apparatus of the invention is conveniently controlled to adjust the implanted restriction device to open and close the food intake passageway. The adjustment device may control the restriction device to steplessly change the restriction of the passageway.

Preferably, the wireless remote control comprises a separate signal transmitter or receiver and a signal receiver or transmitter implanted in the patient. For example, the signal transmitter and signal receiver may transmit and receive a signal in the form of digital pulses, which may comprise a magnetic or electric field. Alternatively, which is preferred, the signal transmitter and signal receiver may transmit and receive an electromagnetic wave signal, a sound wave signal or a carrier wave signal for a remote control signal. The receiver may comprise an implanted control unit for controlling the adjustment device in response to a control signal from the signal transmitter. Any known or conventional signal transmitting or signal receiving means that is suitable for use with a human or mammal patient may be provided as the signal transmitter or signal receiver.

The apparatus of the invention may further comprise an implanted energiser unit for providing energy to energy consuming implanted components of the apparatus, such as electronic circuits and/or a motor for operating the adjustment device. Where a motor is provided the control unit is adapted to power the motor with energy provided by the energiser unit in response to a control signal received from the signal transmitter. The motor may be any type of motor, such as a pneumatic, hydraulic or electric motor and the energiser unit may power the motor with pressurized gas or liquid, or electric energy, depending on the type of motor. Where the motor is an electric motor, it may power pneumatic or hydraulic equipment.

The remote control advantageously comprises wireless energy transfer device for transferring energy from outside the patient's body to energy consuming implantable components of the apparatus. The energy transfer device may comprise said energiser unit is adapted to transform energy from the control signal, as it is transmitted to the signal receiver, into electric energy. Where the operation device comprises a motor the wireless energy transfer device is adapted to directly power the motor with transferred energy.

The energy transferred by the wireless energy transfer device preferably comprises a signal, suitably a wave signal. The energy transferred by the wireless energy transfer device may comprise an electric field or a magnetic field or a combination thereof. The signal may be analog or digital or a combination thereof. The energy transfer device may transfer the energy from the signal into a direct, pulsating direct or alternating current or a combination thereof.

Any of the above mentioned signals may comprise analog or digital pulses. The analog or digital signal may comprise a magnetic field or an electric field or a combination thereof. Where the signal is a wave signal it may comprise an electromagnetic wave signal, a sound wave signal or a carrier wave signal for a remote control signal or a combination thereof. Where a carrier signal is used it may be frequency, amplitude or frequency and amplitude modulated.

The apparatus of the invention may comprise an implantable source of energy for powering the operation device and/or for energizing other energy consuming components of the apparatus, wherein the energy from the source of energy is releasable from outside the patient's body. Furthermore, the apparatus may comprise an energy transmission device for wireless transmission of energy of a first form and an energy transforming device implantable in the patient for transforming the energy of the first form into energy of a second form, to be supplied to the source of energy and/or other implantable energy consuming parts of the apparatus. The energy transforming device may transform the wireless energy directly or indirectly into energy different than the wireless energy for operation of the restriction device. Typically, the energy of the second form is different than the energy of the first form. The function of the energy transmission device may be different from that of the energy transforming device.

An implantable motor or pump for operating the adjustment device may be provided, wherein the energy transmission device may be adapted to transmit wireless energy in the form of a magnetic field or electromagnetic waves or field for direct power of the motor or pump, as the wireless energy is being transmitted. Suitably, the energy transmission device transmits energy by at least one signal separate from the above mentioned control signal.

An implantable stabiliser for stabilising the energy of the first or second form may be provided. Where the energy of the second form comprises electric current, the stabiliser suitably comprises at least one capacitor.

Generally, the source of energy comprises a battery, accumulator, capacitor or a combination thereof.

In accordance with an embodiment of the invention, the apparatus comprises a control device adapted to produce wireless energy for directly powering the operation device and/or for energizing other energy consuming components of the apparatus.

It should be understood that the energy consuming parts of the apparatus for example a motor or pump may be or may not be energised with the unchanged wirelessly transmitted energy as this being transmitted as well as being or not being energised with energy different than the transmitted energy for example transformed into electrical energy but still directly used for energising the energy consuming parts of the apparatus as the transmitted energy is transmitted. Alternatively the energy consuming parts of the apparatus may be energised from a implanted source of energy or storage device, which still may be loaded with wireless energy. In all these aspects it is preferable to be able to wirelessly control the release of energy and get an feedback of the result of the performed function of the device. Direct use of transmitted energy may be unrelaible without a feedback what has happened, has the energy reached it's goal?

Generally, the wireless energy may comprise a wave signal including a sound wave signal, an ultrasound wave signal, an electromagnetic wave signal, an infrared light signal, a visible light signal, an ultra violet light signal, a laser light signal, a micro wave signal, a radio wave signal, an x-ray radiation signal or a gamma radiation signal.

Any of the above mentioned signals may comprise a wave signal including a sound wave signal, an ultrasound wave signal, an electromagnetic wave signal, an infrared light signal, a visible light signal, an ultra violet light signal, a laser light signal, a micro wave signal, a radio wave signal, an x-ray radiation signal or a gamma radiation signal.

The control device may be adapted to produce wireless energy in the form of a train of energy pulses and the energy transfer device may be adapted to intermittently transfer the train of energy pulses for direct use in connection with the energising of the energy consuming components of the apparatus. Alternatively, the control device may be adapted to control the energy transforming device to produce the energy of the second form in said train of energy pulses for direct use in connection with the operation of the adjustment device. The transferred energy preferably comprises electric energy. An implantable capacitor may be provided for producing the train of energy pulses.

Where a capacitor is used in any of the above described embodiments it may have a relatively low capacity, i.e. less than 0,1 µF, in order to be small and suited for implantation.

Where the operation device comprises an implantable motor or pump for operating the adjustment device, the energy transfer device may be adapted to directly power the motor or pump with transferred energy, at the same time as the energy is transferred. Where a pump is used it should not be a plunger type of pump, because a plunger pump is noisy, but may comprise a peristaltic or membrane pump.

As mentioned above the apparatus comprises a wireless remote control for non-invasively controlling the operation device, which preferably is electrically powered. Alternatively, the operation device is powered by magnetic energy, non-magnetic energy, electromagnetic energy, non-electromagnetic energy, kinetic energy, non-kinetic energy, sonic energy, non-sonic energy, thermal energy or non-thermal energy. However, the operation device may be unpowerable by permanent static magnetic energy. Any other kind of energy, such as electric, electromagnetic energy or a moving permanent magnetic energy, may be conceivable for operating the adjustment device. As a result, the implanted restriction device would not be accidentally adjusted if the patient comes close to any permanent magnet. Suitably, the operation device is adapted to non-invasively operate the adjustment device.

The hydraulic operation device may use hydraulic fluid, the viscosity of which changes when the hydraulic fluid is exposed to energy, preferably electric energy, different than thermal energy. However, use of hydraulic fluid of the kind having a viscosity that substantially increases when exposed to heat or a magnetic field, i.e. the hydraulic fluid would not become more viscous when exposed to heat or influenced by magnetic forces, should be avoided, because external heat sources or heat from the body when the patient has fever and external magnetic sources might affect the implanted components of the apparatus.

The adjustment device may be operable to adjust the restriction device to steplessly change the restriction of the food intake passageway. Furthermore, the adjustment device may be adapted to mechanically adjust the restriction device. Alternatively, it may be adapted to hydraulically adjust the restriction device by using hydraulic means, which is devoid of hydraulic fluid of the kind having a viscosity that substantially increases when exposed to heat or a magnetic field.

In accordance with an embodiment of the invention, the apparatus comprises a control device for controlling the restriction device. The control device may comprise an internal programmable control unit implantable in the patient and, possibly an external control unit outside the patient's body for programming the programmable internal control unit. Alternatively, the external control unit may be programmable and wirelessly control the restriction device. The control device may be adapted to produce wireless energy for directly powering the operation device and/or for energizing other energy consuming components of the apparatus.

At least one sensor for sensing at least one physical parameter of the patient may conveniently be implanted in the patient. The sensor may preferably sense as the physical parameter the horizontal position of the patient or may comprise a pressure sensor for sensing the pressure against the restriction device or the stomach or esophagus or other important parameters. The pressure sensor may be any suitable known or conventional pressure sensor such as shown in U.S. patents 5540731, 4846181, 4738267, 4571749, 4407296 or 3939823; or an NPC-102 Medical Angioplasty Sensor.

Either the internal control unit or the external control unit of the control device may suitably control the restriction device to enlarge or close the food intake passageway. For safety the restriction device may enlarge or open the food intake passageway in response to the sensor sensing for example an abnormally high pressure value. The internal control unit may directly control the restriction device in response to signals from the sensor.

Wherever magnetic means is utilized according to the invention it may comprise a permanent magnet and a magnetic material reed switch, or other suitable known or conventional magnetic means.

Where a source of energy is used the control device suitably is operable from outside the patient's body for controlling the source of energy to release energy for use in connection with the operation of the adjustment device, when the adjustment device is implanted. The source of energy may be provided external to the patient's body, and the control device may be adapted to control the external source of energy to release wireless energy for use in connection with the operation of the adjustment device.

The control device may control the source of energy to release magnetic energy, non-magnetic energy, electromagnetic energy, non-electromagnetic energy, kinetic energy, non-kinetic energy, sonic energy, non-sonic energy, thermal energy or non-thermal energy, preferably in a non-invasive manner and for a determined time period and/or in a determined number of energy pulses.

Where the implantable components of the apparatus comprise electrical electrical components they may include at least one or a single voltage level guard. In this case, the electrical components suitably are devoid of any current detector and/or charge level detector. Furthermore, the electrical components may comprise a capacitor or accumulator, wherein the charge and discharge of the capacitor or accumulator is controlled by use of the voltage level guard. As a result, there is no need for any implanted current detector and/or charge level detector for the control of the capacitor, which makes the apparatus simple and reliable.

In accordance with an advantageous embodiment of the invention, the apparatus comprises an implantable switch for directly or indirectly switching the operation of the restriction device. The switch may be operated by the energy supplied by the energy transmission device mentioned above to switch from an off mode, in which the implantable source of energy mentioned above is not in use, to an on mode, in which the source of energy supplies energy for the operation of the restriction device.

In accordance with an alternative embodiment, the above mentioned a remote control may be employed for controlling the implantable source of energy, wherein the switch is operated by the energy supplied by the energy transmission device to switch from an off mode, in which the remote control is prevented from controlling the source of energy and the source of energy is not in use, to a standby mode, in which the remote control is permitted to control the source of energy to supply energy for the operation of the restriction device.

In accordance with another alternative embodiment, the switch is operated by the energy supplied by the implantable energy transforming device mentioned above to switch from an off mode, in which the source of energy is not in use, to an on mode, in which the source of energy supplies energy for the operation of the restriction device.

In accordance with yet another alternative embodiment, the switch is operated by the energy supplied by the energy transforming device to switch from an off mode, in which the remote control is prevented from controlling the source of energy and the source of energy is not in use, to a standby mode, in which the remote control is permitted to control the source of energy to supply energy for the operation of the restriction device.

Suitably, the restriction device is embedded in a soft or gel-like material, such as a silicone material having hardness less than 20 Shore.

The energy transforming device may be designed to be implanted subcutaneously or in the abdomen, thorax or cephalic region of the patient.

The adjustment device may be adapted to adjust the restriction device such that the restriction device provides a predetermined contraction of the food intake passageway that is satisfactory for the patient.

Both the adjustment means and the operation device may be powered and operable in a non-invasive manner.

The restriction means is operable to open and close the food passageway and normally operable to steplessly adjust the restriction of the food passageway. The restriction means is operable to open the food passageway when food should pass and the patient is swallowing and otherwise close the food passage way to prevent reflux of acid from the stomach.

The adjustment device is normally adapted to adjust the restriction device in a non-flux magnetic, non-thermal or non-viscosity changing manner because these gives an unrelaible function of the device. With non-viscosity changing manner should be understood when the adjustment device primary is adjusted with the change of the viscosity of hydraulic fluid.

All the above described various components, such as the motor, pump and capacitor, may be combined in the different embodiments where applicable. Also the various functions described in connection with the above embodiments of the invention may be used in different applications, where applicable. Specifically, the various remote control functions described and all the various methods for supplying energy may be used in any conceivable combination that is apparent to those skilled in the art.

It is here also described a method of treating a human or animal having heartburn and reflux disease, comprising (a) Surgically implanting in the abdomen of the human or animal an adjustable restriction device which restricts a food passageway in the stomach close to the cardia or in the esophagus. And (b) from time to time, adjusting the restriction device so as (i) to enlarge the restricted passageway to allow food to readily pass therethrough into the human's or animal's stomach, or to allow the human or animal to regurgitate, or (ii) to restrict the restricted passageway sufficiently so as to substantially prevent regurgitation of stomach acids and foods into the esophagus. The restriction device comprises a cavity which is expandable and contractable by the supply of hydraulic fluid thereto, wherein (a) is practised in part by implanting in the human or animal a reservoir containing a predetermined amount of hydraulic fluid and connecting the reservoir to the cavity and a hydraulic operation means for distributing fluid from the reservoir to the cavity, and wherein (b) is practised by controlling the hydraulic operation means from a point outside the human's or animal's body without physically penetrating the human's or animal's body.

In accordance with one alternative, the restriction device may comprises a cavity which is expandable and contractable by the supply of hydraulic fluid thereto, wherein (a) is practised in part by subcutaneously implanting in the human or animal an injection port connected to the cavity of the restriction device, and wherein (b) is practiced by injecting fluid through the injection port to expand the cavity to restrict the passageway and by withdrawing fluid from the injection port to contract the cavity to enlarge the passageway.

In accordance with another alternative, the restriction device is acted upon by an adjustment device which mechanically adjusts the restriction of the food passageway; wherein (a) is practised in part by implanting in the human or animal the adjustment device, implanting a reservoir containing a predetermined amount of hydraulic fluid and connecting the reservoir to the cavity, and implanting a hydraulic operation means for distributing fluid from the reservoir to the cavity; and wherein (b) is practised by controlling the hydraulic operation means from a point outside the human or animal's body without physically penetrating the human's or animal's body to control the adjustment device so that the restriction of the food passageway is changed.

In accordance with yet another alternative, (a) is practised by: (i) inflating the human's or animal's abdomen with gas by penetration of the human's or animal's skin, (ii) introducing at least two laparascopic trocars into the abdomen to introduce the restriction device and one or more medical instruments, and then (iii) applying the restriction device on the esophagus or stomach.

It is here described also a further method of treating a human or animal having heartburn and reflux disease, comprising (a) Surgically implanting in the abdomen of the human or animal an adjustable restriction device which restricts a food passageway in the stomach close to the cardia or in the esophagus. And (b) from time to time, adjusting the restriction device so as (i) to enlarge the restricted passageway to allow food to readily pass therethrough into the human's or animal's stomach, or to allow the human or animal to regurgitate, or (ii) to restrict the restricted passageway sufficiently so as to substantially prevent regurgitation of stomach acids and foods into the esophagus.

It is here described also a further method of treating a human or animal having heartburn and reflux disease, comprising the steps of: (a) Laparascopically placing a restriction device of the device through the abdomen or thorax of the human or animal. (b) Placing at least two laparoscopic trocars within the human's or animal's body. (c) Using a dissecting tool inserted through the laparoscopic trocar, dissecting the region of the esophagus or stomach. (d) Introducing the restriction device through the trocars. (e) Placing the restriction device in engagement with the esophagus or the upper part of the stomach to create a restricted stoma. And (f) from time to time, adjusting the restriction device so as (i) to enlarge the restricted stoma to allow food to readily pass therethrough into the human's or animal's stomach, or to allow the human or animal to regurgitate, or (ii) to restrict the restricted stoma sufficiently so as to substantially prevent regurgitation of stomach acids and foods into the esophagus.

The invention is described in more detail in the following with reference to the accompanying drawings, in which
FIGURE 1A-D are block diagrams of four different principal examples of heartburn and reflux disease treatment apparatues. Figures 1B and 1D show embodiments according to the invention.
FIGURE. 2A-D are cross-sectional views of a pump mechanism according to FIGURE 1C, which is designed to pump fluid in opposite directions by mechanically pushing a wall portion in only one direction.
FIGURE 3 is a cross-sectional view of a reservoir having a variable volume controlled by a remote control motor, in accordance with a particular embodiment of the principal embodiment shown in FIGURE 1B or 2B.
FIGURE 4 is a cross-sectional view of a reservoir having a variable volume adjustable by manual manipulation, in accordance with a particular embodiment of the principal embodiment shown in FIGURE 1B or 1D.
FIGURE 5A is a perspective view of a hydraulic, pneumatic or mechanical servo system in accordance with a particular embodiment of the principal embodiment shown in FIGURE 1D.
FIGURE 5B is a cross-sectional view taken along line VB-VB of Fig 5A.
FIGURE 6 is a block diagram illustrating remote control components of the device of the invention;
FIGURE 7 is a schematic view of exemplary circuitry used for the block diagram in FIGURE 4;
FIGURE 8 is a schematic view af a band with a cavity defining a restriction opening for use in accordance with the invention.
FIGURES 9A and 9B are schematic views of a first mechanical restriction device for use not in accordance with the invention;
FIGURES 10A and 10B are schematic views of a second mechanical restriction device for use not in accordance with the invention;
FIGURE 11 is a schematic view of a third mechanical restriction device for use not accordance with the invention;
FIGURE 12A is a schematic front view of a fourth mechanical restriction device for use not in accordance with the invention;
FIGURES 12B and 12C are sectional views along the line A-A of FIGURE 12A;
FIGURES 13A through 17B are five modifications of the examples of FIGURES 12A-12C;
FIGURE 18 illustrates an embodiment of the device in accordance with the invention implanted in a patient and non-invasively controlled by a wireless remote control; and
FIGURE 19 illustrates another embodiment of the device in accordance with the invention implanted in a patient and invasively adjustable.

Referring to the drawing figures, like reference numerals designate identical or corresponding elements throughout the several figures.

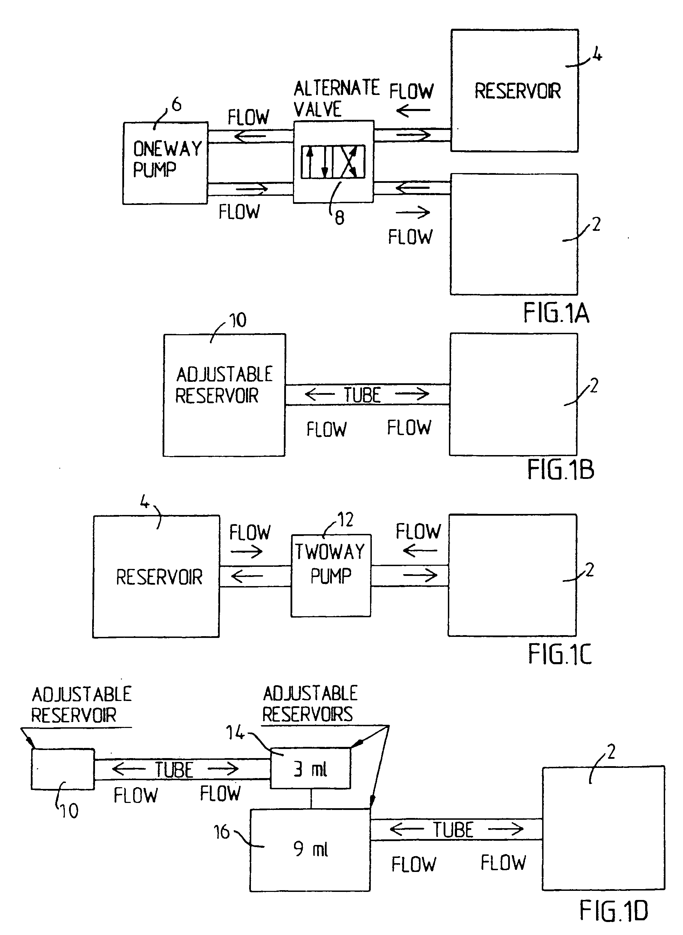

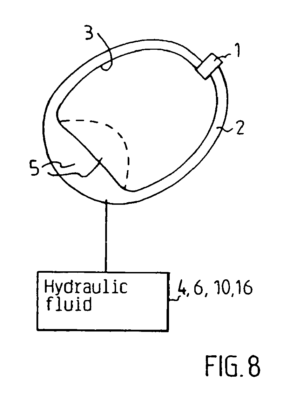

FIGURE 1A-D is a block diagram of four different examples of heartburn and reflux disease devices. FIGURE 1A shows an elongated restriction member in the form of a band 2 forming a loop which defines a restriction opening. The band 2 provides a restricted cross-sectional area of the food passageway in the stomach or esophagus when applied around the esophagus or stomach. FIGURE 1A further shows a separate reservoir 4, a one-way pump 6 and an alternate valve 8. FIGURE 1B shows the band 2 and a fluid supply reservoir 10. FIGURE 1C shows the band 2, a two-way pump 12 and the reservoir 4. FIGURE 1D shows a servo system with a first closed system controlling a second system. The servo system comprises the fluid supply reservoir 10 and a servo reservoir 14. The servo reservoir 14 controls a larger adjustable reservoir 16 which in connection with the band 2 applied around the stomach immediately close to the cardia or around the esophagus varies the volume of a cavity in the band, which in turn varies the restricted cross-sectional area of the food passageway. Such a band 2 forming the restriction opening 3 is illustrated schematically in FIGURE 8. The band 2 comprises an adjustment device having an expandable/contractabe cavity 5 which is expanded or contracted by supplying hydraulic fluid (e.g. from reservoir 4, 6, 10, or 16), and the band 2 may be sutured in place, illustrated schematically at 7 in FIGURE 8.

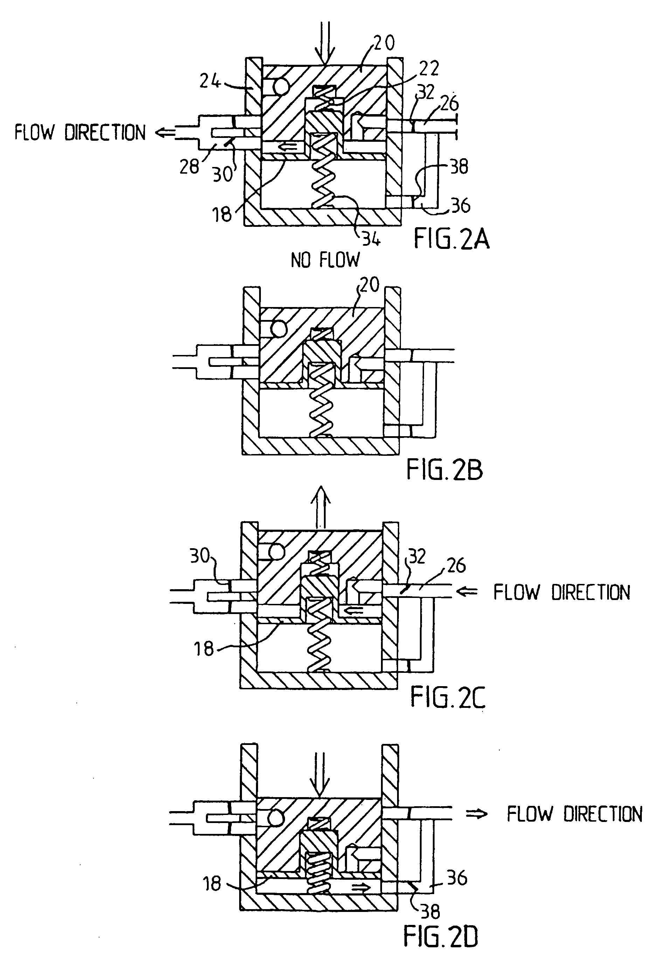

FIGURES 2A-D are cross-sectional views of a pump mechanism adapted to pump fluid in both directions only by mechanically pushing a separate sealing wall portion 18 in one direction. FIGURE 2A shows a piston 20 pushed forwards against a spring 22 towards the wall portion 18 and located in a pump housing 24 conducting fluid from a right upper fluid passage 26 of the housing 24 to a left fluid passage 28 of the housing 24. A main valve 30 is open and a nonreturn valve 32 is closed. FIGURE 2B illustrates the first pump movement in which the piston 20 has moved forwards and reaches the wall portion 18. FIGURE 2C illustrates how the piston 20 moves backwards by the action of the spring 22. The main valve 30 is now closed and the nonreturn valve 32 is open for fluid from the right upper passage 26. FIGURE 1D illustrates how the piston 20 is moved further downwards from its position according to FIGURE 2B while pushing the wall portion 18 downwardly against a second spring 34 that is stronger than spring 22, whereby fluid escapes from a right lower fluid passage 36. When moving the piston 20 backwardly from the position according to FIGURE 2D, fluid enters the left fluid passage 28 and a valve 38 in the lower right fluid passage 36 closes.

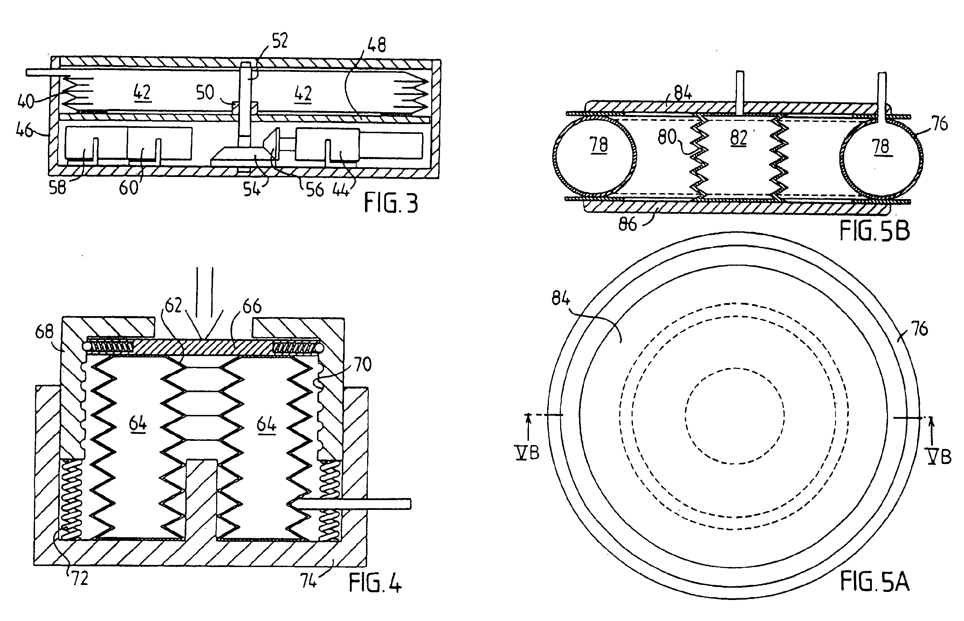

FIGURE 3 is a cross-sectional view of a reservoir 40 defining a chamber 42, the size of which is variable and is controlled by a remote controlled electric motor 44, in accordance with FIGURE 1B or 1D. The reservoir 40 and the motor 44 are placed in a housing 46. The chamber 42 is varied by moving a large wall 48. The wall 48 is secured to a nut 50, which is threaded on a rotatable spindle 52. The spindle 52 is rotated by the motor 44 via an angular gearing, which comprises two conical gear wheels 54 and 56 in mesh with each other. The motor 44 is powered by a battery 58 placed in the housing 46. An signal receiver 60 for controlling the motor 44 is also placed in the housing 46. Alternatively, the battery 58 and the signal receiver 60 may be mounted in a separate place. The motor 44 may also be powered by energy transferred from transmitted signals.

FIGURE 4 is a cross-sectional view of a reservoir 62 defining a chamber 64, the size of which is variable and is controlled by manual manipulation. A gable wall portion 66 of an open ended inner cylindrical housing 68 is adapted to be pushed downwards to fit in a desired locking groove 70 of a plurality of locking grooves 70 on the mantle wall of the cylindrical housing 68, to reduce the size of the chamber 64. The inner cylindrical housing 68 is suspended by springs 72 and is telescopically applied on an outer cylindrical housing 74. When pushing the inner cylindrical housing 68 it moves downwards relative to the outer cylindrical housing 74 causing the gable wall portion 66 to release from the locking groove 70 and move upwards relative to the inner cylindrical housing 68. When the inner housing 68 is moved upwardly by the action of the springs 72 the size of the chamber 64 is increased.

FIGURES 5A and 5B show a servo means comprising a main ring-shaped fluid reservoir 76 defining a chamber 78, the size of which is variable. Centrally positioned in the main ring-shaped reservoir 76 there is a servo fluid reservoir 80 defining a chamber 82, the size of which is variable. The chamber 82 of the servo reservoir 80 is substantially smaller than the chamber 78 of the main reservoir 76. The two reservoirs 76 and 80 are situated between two opposite separate walls 84 and 86, and are secured thereto. When changing the amount of fluid in the servo reservoir 80, the two opposite walls 84,86 are moved towards or away from each other, whereby the size of the chamber 78 of the main reservoir 76 is changed.

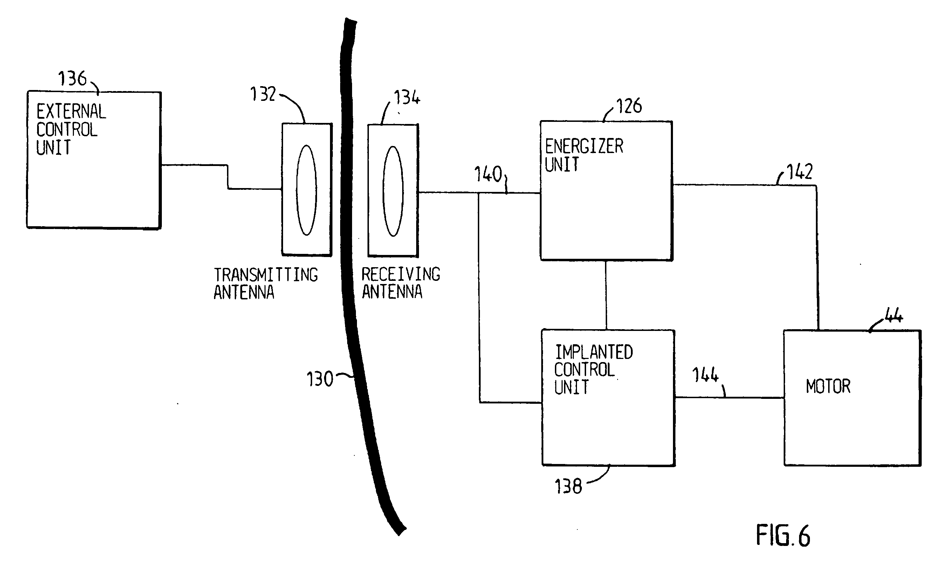

FIGURE 6 shows the basic parts of a remote control system of the device of the invention including the electric motor 44 of the embodiment shown in FIGURE 3. In this case, the remote control system is based on the transmission of electromagnetic wave signals, often of high frequencies in the order of 100 kHz - 1 gHz, through the skin 130 of the patient. In FIGURE 6, all parts placed to the left of the skin 130 are located outside the human's or animal's body and all parts placed to the right of the skin 130 are implanted. Any suitable remote control system may be used.

An external signal transmitting antenna 132 is to be positioned close to a signal receiving antenna 134 implanted close to the skin 130. As an alternative, the receiving antenna 134 may be placed for example inside the abdomen of the patient. The receiving antenna 134 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 132 comprises a coil having about the same size as the coil of the receiving antenna 134 but wound with a thick wire that can handle the larger currents that is necessary. The coil of the transmitting antenna 132 is tuned to the same specific high frequency as the coil of the receiving antenna 134.

An external control unit 136 comprises a microprocessor, a high frequency electromagnetic wave signal generator and a power amplifier. The microprocessor of the control unit 136 is adapted to switch the generator on/off and to modulate signals generated by the generator to send digital information via the power amplifier and the antennas 132,134 to an implanted control unit 138. To avoid that accidental random high frequency fields trigger control commands, digital signal codes are used. A conventional keypad placed on the external control unit 136 is connected to the microprocessor thereof. The keypad is used to order the microprocessor to send digital signals to either increase or decrease the size of the restriction opening defined by the loop of the restriction member 2. The microprocessor starts a command by applying a high frequency signal on the antenna 132. After a short time, when the signal has energized the implanted parts of the control system, commands are sent to increase or decrease the size of the restriction opening of the restriction member 2 in predefined steps. The commands are sent as digital packets in the form illustrated below.

| | | | |
|---|---|---|---|
| Start pattern, 8 bits | Command, 8 bits | Count, 8 bits | Checksum, 8 bits |

The commands are sent continuously during a rather long time period (e.g. about 30 seconds or more). When a new increase or decrease step is desired the Count byte is increased by one to allow the implanted control unit 138 to decode and understand that another step is demanded by the external control unit 136. If any part of the digital packet is erroneous, its content is simply ignored.

Through a line 140, an implanted energizer unit 126 draws energy from the high frequency electromagnetic wave signals received by the receiving antenna 134. The energizer unit 126 stores the energy in a power supply, such as a large capacitor, powers the control unit 138 and powers the electric motor 44 via a line 142.

The control unit 138 comprises a demodulator and a microprocessor. The demodulator demodulates digital signals sent from the external control unit 136. The microprocessor of the control unit 138 receives the digital packet, decodes it and, provided that the power supply of the energizer unit 126 has sufficient energy stored, sends a signal via a signal line 144 to the motor 44 to either increase or decrease the size of the restriction opening of the restriction member 2 depending on the received command code.

Alternatively, the energy stored in the power supply of the energizer unit may only be used for powering a switch, and the energy for powering the motor 44 may be obtained from another implanted power source of relatively high capacity, for example a battery. In this case the switch is adapted to connect the battery to the control unit 138 in an on mode when the switch is powered by the power supply and to keep the battery disconnected from the control unit in a standby mode when the switch is unpowered.

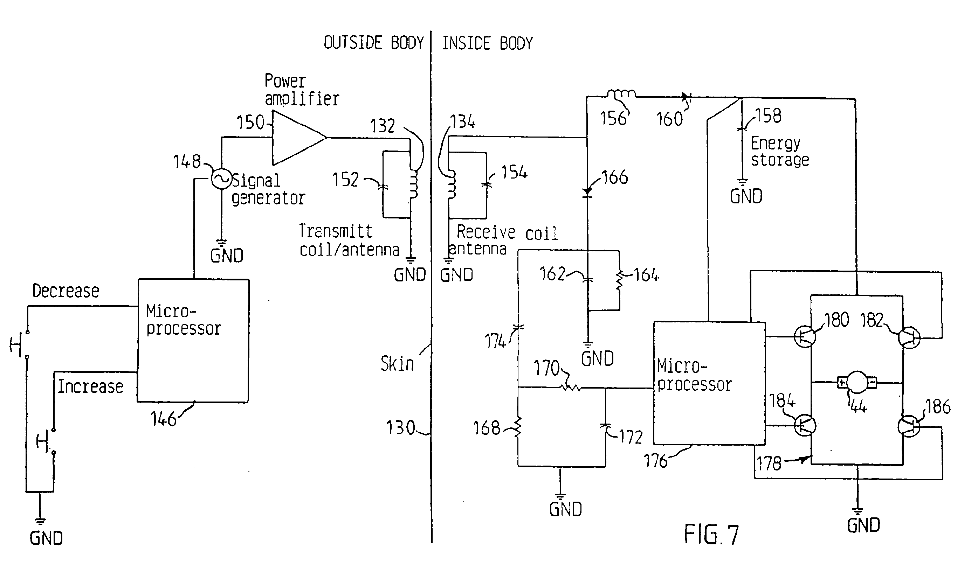

With reference to FIGURE 7, the remote control system schematically described above will now be described in accordance with a more detailed embodiment. The external control unit 136 comprises a microprocessor 146, a signal generator 148 and a power amplifier 150 connected thereto. The microprocessor 146 is adapted to switch the signal generator 148 on/off and to modulate signals generated by the signal generator 148 with digital commands that are sent to implanted components of the device. The power amplifier 150 amplifies the signals and sends them to the external signal transmitting antenna 132. The antenna 132 is connected in parallel with a capacitor 152 to form a resonant circuit tuned to the frequency generated by the signal generator 148.

The implanted signal receiving antenna coil 134 forms together with a capacitor 154 a resonant circuit that is tuned to the same frequency as the transmitting antenna 132. The signal receiving antenna coil 134 induces a current from the received high frequency electromagnetic waves and a rectifying diode 160 rectifies the induced current, which charges a storage capacitor 158. A coil 156 connected between the antenna coil 134 and the diode 160 prevents the capacitor 158 and the diode 160 from loading the circuit of the signal receiving antenna 134 at higher frequencies. Thus, the coil 156 makes it possible to charge the capacitor 158 and to transmit digital information using amplitude modulation.

A capacitor 162 and a resistor 164 connected in parallel and a diode 166 forms a detector used to detect amplitude modulated digital information. A filter circuit is formed by a resistor 168 connected in series with a resistor 170 connected in series with a capacitor 172 connected in series with the resistor 168 via ground, and a capacitor 174, one terminal of which is connected between the resistors 168,170 and the other terminal of which is connected between the diode 166 and the circuit formed by the capacitor 162 and resistor 164. The filter circuit is used to filter out undesired low and high frequencies. The detected and filtered signals are fed to an implanted microprocessor 176 that decodes the digital information and controls the motor 44 via an H-bridge 178 comprising transistors 180,182,184 and 186. The motor 44 can be driven in two opposite directions by the H-bridge 178.

The microprocessor 176 also monitors the amount of stored energy in the storage capacitor 158. Before sending signals to activate the motor 44, the microprocessor 176 checks whether the energy stored in the storage capacitor 158 is enough. If the stored energy is not enough to perform the requested operation, the microprocessor 176 waits for the received signals to charge the storage capacitor 158 before activating the motor 44.

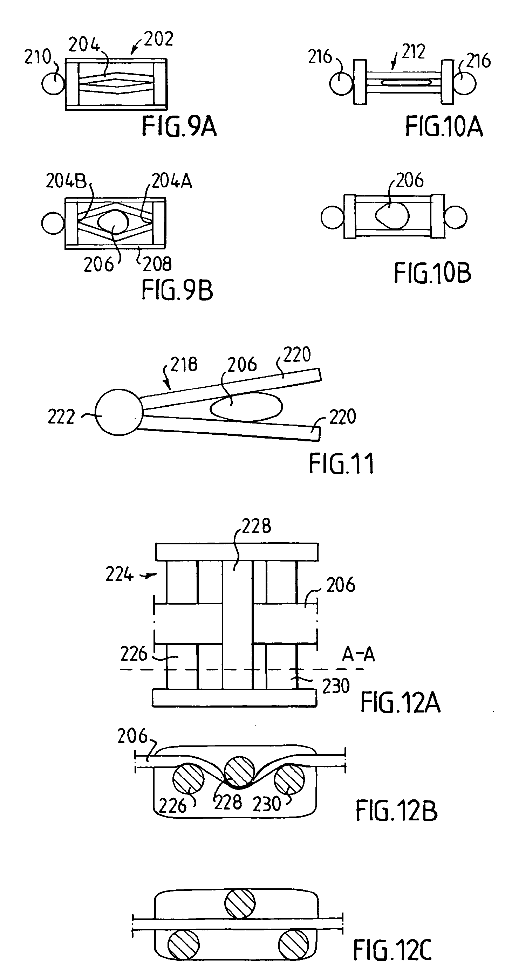

FIGURES 9A and 9B show an example of a device comprising a restriction device 202 having an elongated flexible restriction member 204, such as a belt, a cord or the like. The flexible member 204 extends in a loop around the esophagus 206 (or stomach). (Alternatively, the flexible member 204 may comprise two separate parts on opposite sides of the esophagus.) One portion 204A of member 204 is attached to a frame 208 and another portion 204B of member 204 opposite portion 204A in the loop of the flexible member 204 is connected to an adjustment device 210, which is fixed to the frame 208. The adjustment device 210 pulls the flexible member 204 in the direction from portion 204A to squeeze the esophagus between two opposite lengths of the flexible member 204 to thereby decrease the cross-sectional area in the esophagus (or stomach), see FIGURE 96A, and releases the esophagus from the flexible member 204 to thereby increase the cross-sectional area in the esophagus 206, see FIGURE 9B.

FIGURES 10A and 10B show an example of a device comprising a restriction device 212 having two plate or bar elements 214 on opposite sides of the esophagus 206 (or stomach). An adjustment device 216 moves the elements 212 in parallel towards each other to squeeze the esophagus 206 between the elements 212 to thereby decrease the cross-sectional area in the esophagus, see FIGURE 10A, and moves the elements 212 away from each other to increase the cross-sectional area in the esophagus 206, see FIGURE 10B.

FIGURE 11 shows an example comprising a restriction device 218 having two articulated clamping elements 220 positioned on opposite sides of the esophagus 206 (or stomach). An adjustment device 222 moves the clamping elements 220 toward each other to clamp the esophagus 206 between the clamping elements 220 to thereby decrease the cross-sectional area in the esophagus 206, and moves the clamping elements 420 away from each other to release the esophagus 206 from the clamping elements 220 to thereby increase the cross-sectional area in the esophagus 206.

FIGURES 12A, 12B and 12C show an example comprising a restriction device 224 having three bending members in the form of cylindrical rollers 226, 228 and 230 displaced relative one another in a row along the esophagus 206 (or stomach) and positioned alternately on opposite sides of the esophagus 206. (Alternatively, each roller 226, 228 and 230 may take the shape of an hour-glass.) An adjustment device 232 moves the two outer rollers 226,230 laterally against the esophagus 206 in one direction and the intermediate roller 228 against the esophagus 206 in the opposite direction to bend the esophagus to thereby decrease the cross-sectional area in the esophagus 206, see FIGURE 12B. To increase the cross-sectional area in the esophagus 206 the adjustment device 232 moves the rollers 226-230 away from the esophagus 206 to release the latter from the rollers 226-230, see FIGURE 12C.

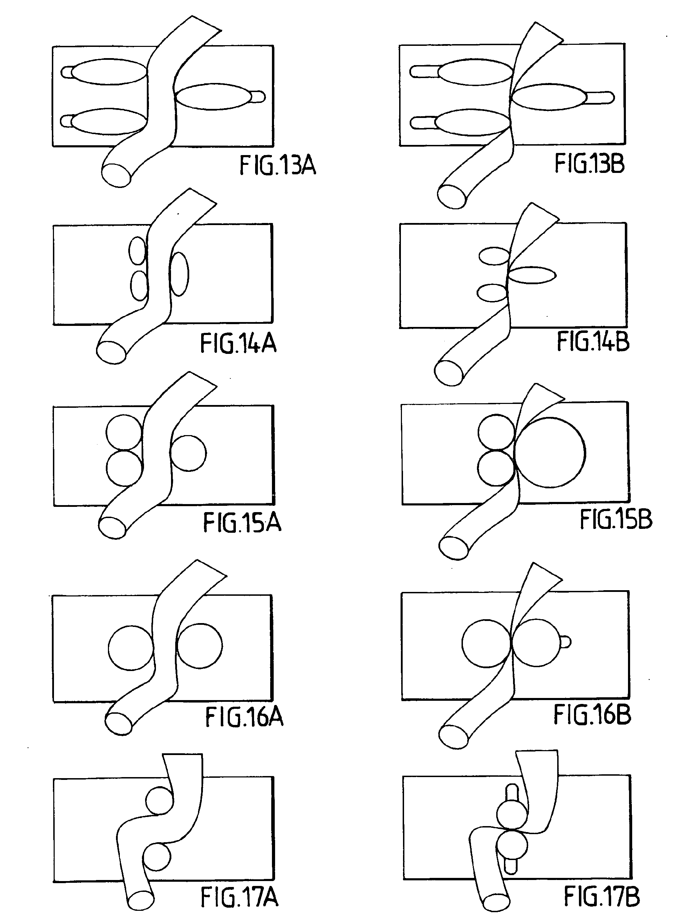

FIGURES 13A through 17B schematically illustrates modifications of the above example according to FIGURES 12A-12C. Thus, FIGURES 13A and 13B show an example similar to that of FIGURES 12A-12C except that the bending members are oval and not rotatable. FIGURES 14A and 14B show an example similar to that of FIGURES 13A and 13B except that the oval bending members are rotatable to release the esophagus (or stomach), see FIGURE 14A, and squeeze the esophagus, see FIGURE 14B. FIGURES 15A and 15B show an example similar to that of FIGURES 12A-12C except that the intermediate roller has a changeable diameter to release the esophagus (or stomach), see FIGURE 15A, and squeeze the esophagus, see FIGURE 15B. FIGURES 16A and 16B show an example similar to that of FIGURES 10A-10C except that the elements are replaced by two cylindrical rollers positioned on opposite sides of the esophagus. Finally, FIGURES 17A and 17B show an example substantially similar to that of FIGURES 16A and 16B except that the restriction device is turned 90° to form a S-shaped curvature of the esophagus.

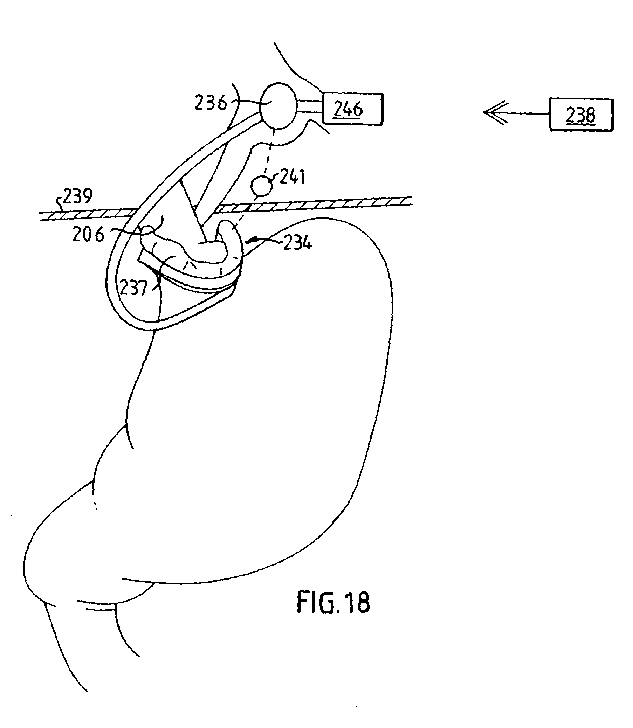

FIGURE 18 illustrates an embodiment of the heartburn and reflux disease treatment apparatus of the invention implanted in a patient. Thus, an assembly of the device implanted in the patient comprises an adjustable restriction device 234 engaging the esophagus 206 close to the cardia, an adjustment device (which may include an inflatable cavity in the restriction device) for adjusting the restriction device, and a unit 236 which includes a hydraulic operation means (which may include a pump) for operating the adjustment device and a fluid reservoir for supplying fluid to the operation means. The restriction device 234 is provided with a soft support member 237, which abuts upwardly against the diaphragm 239 of the patient. A wireless remote control of the device comprises an external signal transmitter 238 and an implanted signal receiver 240, which includes a control unit for controlling the adjustment device of the implanted assembly in response to a control signal from the transmitter 238. The signal receiver 240 further includes an energizer unit which transforms energy from the control signal transmitted by the transmitter 238 into electric energy for energy consuming implanted components of the device.

A pressure sensor 241 is implanted for sensing the pressure on the restriction device 234. The control unit of the signal receiver 240 controls the adjustment device to release the restriction device 234 in response to the pressure sensor 241 sensing an abnormal high pressure.

The embodiment according to FIGURE 18 is particularly suited for patients that require regular adjustments of the restriction device during the day.

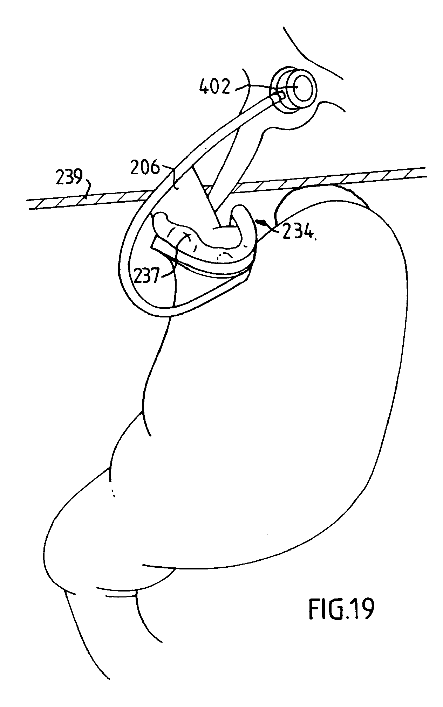

FIGURE 19 illustrates another embodiment of the heartburn and reflux disease treatment apparatus of the invention implanted in a patient. In this embodiment the restriction device 234 is provided with an expandable cavity 237, whereby the size of the restricted cross-sectional area of the food passageway is reduced upon expansion of the cavity and increased upon contraction of the cavity. An injection port 402 is implanted subcutaneously in the patient for transcutaneously adding fluid to and withdrawing fluid from the cavity of the restrictio device 234 by the use of an injection needle. The embodiment according to FIGURE 19 is particularly suited for patients that do not require frequent adjustments of the restriction device 234.

There are a number of conceivable alternative embodiments of the invention that give the same result as the abovedescribed embodiments. For example, the microprocessor of the external and implanted, respectively, control units may be replaced by discrete components. The power amplifier of the external control unit may be omitted if the signals generated by the signal generator are strong enough. Therefore, the invention is to be accorded the broadest interpretation of the appended claims to encompass all equivalent structures and assemblies.

One further advantage with this invention is that there may be a night button on the remote control setting the adjustment device in a position with a larger stoma diameter during the night, thus avoiding vomiting or nausa.

## Claims

1. A heartburn and reflux disease treatment device, comprising an adjustable restriction means (2) adapted to engage the stomach close to the cardia or esophagus of a patient to form a restricted food passageway in the stomach or esophagus, comprising an implantable adjustment means (5) for post-operative adjusting the restriction means (2) to enlarge and restrict the food passageway, when the restriction means is implanted in the patient, an implantable hydraulic operation device (6, 8; 10; 12; 10, 14; 76-86) for operating the adjustment means and a reservoir (4;10;16) implantable in the patient and containing hydraulic fluid, wherein the adjustment means comprises an expandable cavity (5) in the restriction member (2), the food passageway being restricted upon expansion of the cavity and enlarged upon contraction of the cavity, and the hydraulic operation device (6, 8; 10; 12; 10, 14; 76-86) is adapted to distribute hydraulic fluid from the reservoir (4;10;16) to expand the cavity, and to distribute hydraulic fluid from the cavity to the reservoir to contract the cavity, wherein the reservoir (4;10;16) contains a predetermined amount of hydraulic fluid and, wherein the reservoir (10) defines a chamber (42;64) for said predetermined amount of fluid and the operation device is adapted to change the size of the chamber, and wherein the operation device is adapted to change the size of the chamber in response to the pressure in the reservoir.

2. An apparatus according to claim 1, wherein the operation device comprises first and second wall portions (48;66) of the reservoir (10) and is adapted to provide relative displacement between the first and second wall portions of the reservoir, in order to change the volume of the chamber (42; 64).

3. An apparatus according to claim 1, wherein the operation device comprises a pressure controlled hydraulic operation device.

4. An apparatus according to claim 2, wherein the first and second wall portions of the reservoir (10) are displaceable relative to each other by magnetic means, hydraulic means, or electric control means, or a combination thereof.

5. An apparatus according to claim 2, wherein the operation device is adapted to distribute fluid from the reservoir (10) to the cavity (5) of the restriction device (2) in response to a predetermined first displacement of the first wall portion (48;66) of the reservoir relative to the second wall portion of the reservoir and to distribute fluid from the cavity to the reservoir in response to a predetermined second displacement of the first wall portion relative to the second wall portion.

6. An apparatus according to any of claims 1-5, wherein the operation device comprises a servo means.

7. An apparatus according to any of claims 2, 4 or 5 wherein the operation device comprises a reverse servo.

8. An apparatus according to claim 1, wherein the operation device comprises a servo means operatively connected to the adjustment device.

9. An apparatus according to claim 1 or 8, wherein the operation device is powered.

10. An apparatus according to claim 1 or 8, wherein the operation device is manually operated.

11. An apparatus according to claim 8 or 9, wherein the servo means comprises a motor, preferably an electric motor.

12. An apparatus according to claim 11, wherein the motor is reversible.

13. An apparatus according to claim 11 or 12, further comprising a gearing connected between the motor and the adjustment device.

14. An apparatus according to any of claims 8-10, further comprising an implantable reservoir (4;10;16) defining a chamber for hydraulic fluid, wherein the operation device is adapted to operate the adjustment device (5) by using the hydraulic fluid of the reservoir.

15. An apparatus according to claim 14, wherein the reservoir (4; 10; 16) contains a predetermined amount of hydraulic fluid.

16. An apparatus according to any of claims 8-10 or 14-15 wherein the servo means comprises a reverse servo.

17. An apparatus according to claims 14 and 16, wherein the the reservoir (204) comprises first and second wall portions and the reverse servo is adapted to provide relative displacement between the first and second wall portions of the reservoir, in order to change the volume of the reservoir.

18. An apparatus according to any of claims 8-17, wherein the servo means comprises a pressure controlled servo means and
further comprising an alarm adapted to generate an alarm signal in response to the lapse of a predetermined time period during which the pressure controlling the servo means exceeds a predetermined high value.

19. An apparatus according to claim 16, wherein the reverse servo comprises magnetic means, electric means or manual manipulation means or a combination thereof.

20. An apparatus according to claim 17, wherein the reverse servo comprises hydraulic means (10,14, 76-86).

21. An apparatus according to claim 20, wherein the reverse servo further comprises a servo reservoir defining a chamber containing servo fluid, and the operation device comprise first and second wall portions of the servo reservoir, which are displaceable relative to each other to change the volume of the chamber of the servo reservoir.

22. An apparatus according to claim 21, wherein the first and second wall portions of the servo reservoir are displaceable relative to each other by magnetic means, hydraulic means, or electric control means.

23. An apparatus according to claim 20, wherein the reverse servo comprises a servo reservoir (14) and a fluid supply reservoir (10) connected in a closed system and containing a further predetermined amount of fluid.

24. An apparatus according to claim 23, wherein the fluid supply reservoir (10) defines a chamber for the further predetermined amount of fluid and the hydraulic operation device is adapted to change the volume of the chamber and thereby control the amount of fluid in the servo reservoir (14).

25. An apparatus according to claim 24, wherein the fluid supply reservoir (10) comprises first and second wall portions, which are displaceable relative to each other to change the volume of the chamber of the fluid supply reservoir.

26. An apparatus according to claim 25, wherein the fluid supply reservoir (10) increases the amount of fluid in the servo reservoir (14) in response to a predetermined first displacement of the first wall portion of the fluid supply reservoir relative to the second wall portion of the fluid supply reservoir and decreases the amount of fluid in the servo reservoir in response to a predetermined second displacement of the first wall portion of the fluid supply reservoir relative to the second wall portion of the fluid supply reservoir.

27. An apparatus according to claim 1, further comprising an injection port subcutaneously implantable in the patient and in fluid communication with the chamber.

28. An apparatus according to claim 27, wherein the injection port is integrated in the reservoir.

29. An apparatus according to claim 1 or 8, wherein the restriction means comprises an elongated restriction member and forming means for forming the restriction member into at least a substantially closed loop around the esophagus or stomach, the loop defining a restriction opening, whereby the adjustment means is adapted to adjust the restriction member in the loop to change the size of the restriction opening.

30. An apparatus according to claim 29, wherein the forming means forms the restriction member (2) into a loop having a predetermined size or a size selected from several predetermined sizes.

31. An apparatus according to claim 29, wherein the adjustment device is adapted to change the size of the restriction opening such that the outer circumferential confinement surface of the restriction member is changed.

32. An apparatus according to claim 29, wherein the adjustment device is adapted to change the size of the restriction opening such that the outer circumferential confinement surface of the restriction member is unchanged.

33. An apparatus according to claim 29, wherein the restriction member is non-inflatable, and the adjustment device is adapted to adjust the restriction member in said loop.

34. An apparatus according to claim 22, wherein the adjustment device mechanically adjusts the restriction member.

35. An apparatus according to claim 33, wherein the adjustment device hydraulically adjusts the non-inflatable restriction member.

36. An apparatus according to claim 34 or 35, wherein the elongated restriction member (204) is flexible, and the adjustment means (210) is adapted to pull a first portion (204A) of the flexible restriction member from a second portion (204B) of the flexible restriction member opposite the first portion in the loop to squeeze the esophagus (206) or stomach between two opposite lengths of the elongated flexible restriction member to restrict the passageway, and to release the esophagus or stomach from the flexible restriction member to enlarge the passageway.

37. An apparatus according to any of the preceding claims, further comprising a wireless remote control (44,126,132-144) for non-invasively controlling the hydraulic operation device (6, 8; :10; 12; 10, 14; 76-86).

38. An apparatus according to claim 37, wherein the remote control comprises an external wireless hand-held remote control unit which is manually operable by the patient to control the restriction device change the restriction of the food passageway.

39. An apparatus according to claim 37, wherein the remote control (44,126,132-144) comprises an external signal transmitter (132,136), receiver or transceiver and a signal receiver (134,138), transmitter or transceiver implantable in the patient.

40. An apparatus according to claim 39, wherein the signal receiver (134,138) and/or transmitter comprises a control unit (138) adapted to control the operation device (6, 8; 10; 12; 10, 14; 76-86) in response to a control signal received from the signal transmitter (132, 136).

41. An apparatus according to claim 3, further comprising an alarm adapted to generate an alarm signal in response to the lapse of a predetermined time period during which the pressure controlling the hydraulic operation device exceeds a predetermined high value.

42. An apparatus according to claim 40, further comprising an implantable energizer unit (136) for providing energy to energy consuming implantable components of the apparatus.

43. An apparatus according to claim 1 or 8 or 42, wherein the operation device comprises a motor (44) for operating the adjustment device.

44. An apparatus according to claims 42 and 43, wherein the control unit (138) is adapted to power the motor (44) with energy provided by the energizer unit (136) in response to a control signal received from the signal transmitter (132,136).

45. An apparatus according to claim 37, wherein the remote control (44, 126, 132-144) comprises wireless energy transfer means for transferring energy from outside the patient's body to energy consuming implantable components of the apparatus.

46. An apparatus according to claim 42 and 45, wherein the energy transfer means comprises an implantable energizer unit (126), which is adapted to transform energy from the control signal, as it is transmitted to the signal received (134,138), into electric energy.

47. An apparatus according to claim 45, wherein the operation device (6; 8; 10; 12; 10, 14; 76-86) comprises a motor (44), and the wireless energy transfer means is adapted to directly power the motor with transferred energy.

48. An apparatus according to claim 46 or 47, wherein the energy transferred by the wireless energy transfer means comprises a signal.

49. An apparatus according to claim 48, wherein the signal comprises a wave signal.

50. An apparatus according to claim 46 or 47; wherein the energy transferred by the wireless energy transfer means comprises an electric field or a magnetic field or a combination thereof.

51. An apparatus according to claim 48, wherein the signal is analog or digital or a combination thereof.

52. An apparatus according to claim 39 or 40, wherein the signal transmitter (132,136) and signal receiver (134,138) are adapted to transmit and receive an analog or digital signal or a combination thereof.

53. An apparatus according to claim 51 or 52, wherein the signal comprises analog or digital pulses.

54. An apparatus according to any of claims 51-53, wherein the analog or digital signal comprises a magnetic field or an electric field or a combination thereof.

55. An apparatus according to claim 39 or 40, wherein the signal transmitter (132,136) and signal receiver (134,138) are adapted to transmit and receive a wave signal.

56. An apparatus according to claim 48 or 55, wherein the wave signal comprises an electromagnetic wave signal, a sound wave signal or a carrier wave signal for a remote control signal or a combination thereof.

57. An apparatus according to claim 56, wherein the carrier signal is frequency, amplitude or frequency and amplitude modulated.

58. An apparatus according to claim 48 or 51 wherein the energy transfer means transfers the energy from the signal into a direct, pulsating direct or alternating current or a combination thereof.

59. An apparatus according to claim 37, wherein the remote control (44, 126, 132-144) is capable of obtaining information related to important parameters of the apparatus from iside the patient's body and of commanding the adjustment device (5) to adjust the restriction device (2) in response to obtained information.

60. An apparatus according to claim 37, wherein the remote control is capable of obtaining information related to the food passageway in the stomach or esophagus and of commanding the adjustment means to adjust the restriction means in response to obtained information.

61. An apparatus according to any of the claims 1 or 8, further comprising an implantable source of energy for powering the operation device and/or for energizing other energy consuming components of the apparatus, wherein the energy from the source of energy is releasable from outside the patient's body.

62. An apparatus according to any of the claims 1 or 8, further comprising an energy transmission device for wireless transmission of energy.

63. An apparatus according to claim 61 and 62, wherein the energy transmission device transmits energy of a first form, and further comprising an energy transforming device implantable in the patient for transforming the energy of the first form into energy of a second form, to be supplied to the source of energy and/or other implantable energy consuming parts of the apparatus.

64. An apparatus according to claim 63, wherein the energy of the second form is different than the energy of the first form.

65. An apparatus according to claim 63, wherein the energy transmission device functions different from the energy transforming device.

66. An apparatus according to claim 62, further comprising an implantable motor or pump for operating the adjustment device, wherein the energy transmission device is adapted to transmit wireless energy in the form of an magnetic field or electromagnetic waves or field for direct power of the motor or pump, as the wireless energy is being transmitted.

67. An apparatus according to claim 40 and 62, wherein the energy transmission device transmits energy by at least one signal separate from the control signal.

68. An apparatus according to claim 63, further comprising an implantable stabiliser for stabilising the energy of the first or second form.

69. An apparatus according to claim 68, wherein the energy of the second form comprises electric current and the stabiliser comprises at least one capacitor.

70. An apparatus according to any of claims 61,63-65, wherein the source of energy comprises a battery, accumulator, capacitor or a combination thereof.

71. An apparatus according to any of the claims 1 or 8, further comprising a control device adapted to produce wireless energy for directly powering the operation device and/or for energizing other energy consuming components of the apparatus.

72. An apparatus according to any of the claims 1 or 8, further comprising an implantable energy transforming device for transforming wireless energy directly or indirectly into energy different than the wireless energy for operation of the restriction device.

73. An apparatus according to claim 71 or 72, wherein the wireless energy comprises a wave signal including a sound wave signal, an ultrasound wave signal, an electromagnetic wave signal, an infrared light signal, a visible light signal, an ultra violet light signal, a laser light signal, a micro wave signal or a radio wave signal.

74. An apparatus according to any of the claims 1 or 8, further comprising an energy transfer means (22,326,332-344) for wireless transfer of energy from outside the patient's body to the operation device or adjustment device and/or other energy consuming implantable components of the apparatus.

75. An apparatus apparatus according to claim 71, wherein the control device is adapted to produce wireless energy in the form of a train of energy pulses.

76. An apparatus according to claim 45 or 74, wherein the energy transfer means is adapted to intermittently transfer the energy in the form of a train of energy pulses for direct use in connection with the energising of the energy consuming components of the apparatus.

77. An apparatus according to claim 71 and 63 wherein the control device is adapted to control the energy transforming device to produce the energy of the second form in a train of energy pulses for direct use in connection with the operation of the adjustment device.

78. An apparatus according to claim 76 or 77, wherein the energy transfer device is adapted to transfer electric energy, and further comprising an implantable capacitor for producing the train of energy pulses.

79. An apparatus according to claim 78 or 69, 70, wherein the capacitor has a capacity less than 0,1 µF.

80. An apparatus according to claim 74, further comprising an implantable motor (22) or pump for operating the adjustment device (12;52;66;90,92;104;110), wherein the energy transfer means is adapted to directly power the motor or pump with transferred energy.

81. An apparatus according to claim 66 or 80, wherein the pump is not a plunger type of pump.

82. An apparatus according to any of claims 38-81, further comprising a wireless remote control (44, 126, 132-144) for non-invasively controlling the operation device (6, 8; 10; 12; 10, 14; 76-86).

83. An apparatus according to any one of the preceding claims, wherein the operation device is electrically powered.

84. An apparatus according to any of the claims 1, 8, or 66, wherein the operation device is unpowerable by static permanent magnetic energy.

85. An apparatus according to any of the claims 1 or 8, wherein the operation device is adapted to non-invasively operate the adjustment device.

86. An apparatus according to any of the claims 1 or 8, wherein the restriction means is operable to steplessly adjust the restriction of the food passageway.

87. An apparatus according to claim 1 or 8, wherein the operation device comprises a hydraulic operation device which uses hydraulic fluid, the viscosity of which changes when the hydraulic fluid is exposed to energy different than thermal energy.

88. An apparatus according to claim 83, wherein the viscosity of the hydraulic fluid changes when the fluid is exposed to electric energy.

89. An apparatus according to claim 1 or 8, further comprising an adjustment device for adjusting the restriction device to change the restriction of the food intake passageway, wherein the adjustment device is adapted to mechanically adjust the restriction device, or adapted to hydraulically adjust the restriction device by using hydraulic means which is devoid of hydraulic fluid of the kind having a viscosity that substantially increases when exposed to heat or a magnetic field.

90. An apparatus according to claim 1 or 8, further comprising a control device for controlling the restriction device.

91. An apparatus according to claim 90, wherein the control device comprises an internal control unit implantable in the patient for controlling the restriction device.

92. An apparatus according to claim 91, wherein the internal control unit is programmable.

93. An apparatus according to claim 92, wherein the control device comprises an external control unit outside the patient's body, the implantable internal control unit being programmable by the external control unit.

94. An apparatus according to claim 90, wherein the control device comprises an external control unit outside the patient's body for wirelessly controlling the restriction device.

95. An apparatus according to claim 94, wherein the external control unit is programmable.

96. An apparatus according to any one of the preceding claims, further comprising at least one implantable sensor for sensing at least one physical parameter of the patient.

97. An apparatus according to claim 96, wherein the sensor is adapted to directly or indirectly sense as the physical parameter the horizontal position of the patient. /2

98. An apparatus according to claim 96, wherein the sensor comprises a pressure sensor for directly or indirectly sensing as the physical parameter the pressure against the restriction device or part of the human body.

99. An apparatus according to claim 98, wherein the restriction means is adapted to enlarge the food passageway in the stomach or esophagus in response to the pressure sensor sensing a predetermined pressure.

100. An apparatus according to any one of claims 96-99, further comprising a control device for controlling the restriction device in response to signals from the sensor.

101. An apparatus according to claim 100, wherein the control device comprises an internal control unit implantable in the patient and directly controlling the restriction device in response to signals from the sensor.

102. An apparatus according to claim 101, wherein the control device comprises an external control unit outside the patient's body for controlling the restriction device in response to signals from the sensor.

103. An apparatus according to claim 101, wherein the control device comprises an external control unit outside the patient's body for manually controlling the restriction device in response to information from the sensor.

104. An apparatus according to claim 90, further comprising an implantable source of energy, wherein the control device is operable from outside the patient's body for controlling the source of energy to release energy for use in connection with the operation of the prosthesis, when the prosthesis is implanted.

105. An apparatus according to claim 104, wherein the source of energy is intended to be external to the patient's body, and the control device is adapted to control the external source of energy to release wireless energy for use in connection with the operation of the prosthesis.

106. An apparatus according to claim 104, wherein the control device controls the source of energy to release magnetic energy, non-magnetic energy, electromagnetic energy, non-electromagnetic energy, kinetic energy, non-kinetic energy, sonic energy, non-sonic energy, thermal energy or non-thermal energy.

107. An apparatus according to claim 104, wherein the control device controls the source of energy to release energy for a determined time period.

108. An apparatus according to claim 104 or 104 and 75, wherein the control device controls the source of energy to release energy in a determined number of energy pulses.

109. An apparatus according to claim 104, wherein the control device is adapted to control the source of energy to release energy in a non-invasive manner.

110. An apparatus according to any of the claims 1 or 8, further comprising implantable electrical components including at least one voltage level guard.

111. An apparatus according to any of the claims 1 or 8, further comprising implantable electrical components including a single voltage level guard.

112. An apparatus according to claim 110 or 111, wherein the electrical components are devoid of any current detector and/or charge level detector.

113. An apparatus according to any of claims 61, 78, 63, 69 or 70 and 110-112, further comprising an implantable capacitor or accumulator, wherein the charge or discharge of the capacitor or accumulator is controlled by use of the voltage level guard.

114. An apparatus according to claim 40, 46 or 67, wherein the control signal comprises a wave signal including a sound wave signal, an ultrasound wave signal, an electromagnetic wave signal, an infrared light signal, a visible light signal, an ultra violet light signal, a laser light signal, a micro wave signal, or a radio wave signal.

115. An apparatus according to any of the preceding claims, further comprising a switch implantable in the patient for directly or indirectly switching the operation of the restriction device.

116. An apparatus according to claim 115 and 62, further comprising a source of energy implantable in the patient for supplying energy for the operation of the restriction device, wherein the switch is operated by the energy supplied by the energy transmission device to switch from an off mode, in which the source of energy is not in use, to an on mode, in which the source of energy supplies energy for the operation of the restriction device.

117. An apparatus according to claim 115 and 62, further comprising a source of energy implantable in the patient for supplying energy for the operation of the restriction device, and a remote control for controlling the supply of energy of the implantable source of energy, wherein the switch is operated by the energy supplied by the energy transmission device to switch from an off mode, in which the remote control is prevented from controlling the source of energy and the source of energy is not in use, to a standby mode, in which the remote control is permitted to control the source of energy to supply energy for the operation of the restriction device.

118. An apparatus according to claim 115 and 63, further comprising a source of energy implantable in the patient for supplying energy for the operation of the restriction device, wherein the switch is operated by the energy supplied by the energy transforming device to switch from an off mode, in which the source of energy is not in use, to an on mode, in which the source of energy supplies energy for the operation of the restriction device.

119. An apparatus according to claim 115 and 63, further comprising a source of energy implantable in the patient for supplying energy for the operation of the restriction device, and a remote control for controlling the supply of energy of the implantable source of energy, wherein the switch is operated by the energy supplied by the energy transforming device to switch from an off mode, in which the remote control is prevented from controlling the source of energy and the source of energy is not in use, to a standby mode, in which the remote control is permitted to control the source of energy to supply energy for the operation of the restriction device.

120. An apparatus according to any of the claims 1 or 8, wherein the prosthesis is operable to perform a reversible function.

121. An apparatus according to claim 42 or 120, further comprising a reversing device implantable in the patient for reversing the function performed by the prosthesis.

122. An apparatus according to claim 121, wherein the control device controls the reversing device to reverse the function performed by the prosthesis.

123. An apparatus according to claim 121, wherein the reversing device comprises hydraulic means including a valve for shifting the flow direction of a flowing fluid in the hydraulic means.

124. An apparatus according to claim 121, wherein the reversing device comprises a mechanical reversing device.

125. An apparatus according to claim 121, wherein the reversing device comprises a switch.

126. An apparatus according to claim 125, wherein the switch of the reversing device is operable by the released energy.

127. An apparatus according to claim 126, wherein the control device controls the operation of the switch of the reversing device by shifting polarity of the released energy supplied to the switch.

128. An apparatus according to claim 121, wherein the operation device comprises a motor, and the reversing device reverses the motor.

129. An apparatus according to any of the preceding claims, wherein the restriction device is embedded in a soft or gel-like material.

130. An apparatus according to claim 129, wherein the restriction device is embedded in a silicone material having hardness less than 20 Shore.

131. An apparatus according to claim 63, wherein the energy transforming means or device is designed to be implanted subcutaneously or in the abdomen, thorax or cephalic region of the patient.

132. An apparatus according to any of the preceding claims, wherein the adjustment device is adapted to adjust the restriction device such that the restriction device provides a predetermined contraction of the food passageway that is satisfactory for the patient.

133. An apparatus according any of the claims 1 or 8, wherein the adjustment device is adapted to adjust the restriction device in a non-flux magnetic, non-thermal or non-viscosity changing manner.

134. An apparatus according to claim 1, wherein the adjustment means comprises an expandable cavity in the restriction means, the food passageway is restricted upon expansion of the cavity and enlarged upon contraction of the cavity, and the hydraulic operation means comprises an injection port implantable subcutaneously in the patient for transcutaneously adding fluid to and withdrawing fluid from the cavity.

135. An apparatus according to claim 1 or 8, wherein the adjustment means comprises an hydraulic operation means, and the hydraulic operation means comprises an injection port implantable subcutaneously in the patient for transcutaneously adding fluid to and withdrawing fluid to the hydraulic operation means.

136. An apparatus according to claim 135, wherein the injection port is used only for calibration purposes and the adjustment device is further able to increase the foood restriction opening when food should pass when the patient is eating and swallows food.

137. An apparatus according to claim 136 and 8, wherein the servo is hydraulically operated and, wherein the injection port is used only for calibration purposes of the servo.

138. An apparatus according to claim 2, wherein the first and second wall portions (66) of the reservoir are designed to be displaceable relative to each other by manual manipulation thereof.

139. An apparatus according to any of the preceeding claims, further comprising at least one holding device implantable in the patient for holding the esophagus or stomach in a position where the left and right crus muscles are located, to prevent the region of the cardia from moving cranial through the diaphragm muscle.

140. An apparatus according to claim 139, wherein the holding device is placed in engagement with the food restriction apparatus.

141. An apparatus according to claim 1 or 8, wherein the restriction means is operable to open and close the food passageway.

142. An apparatus according to claim 141, wherein the restriction means is operable to open the food passageway when food should pass and the patient is swallowing and otherwise close the food passage way to prevent reflux of acid from the stomach.

143. An apparatus according to claim 1 or 8, wherein the restriction means is operable to steplessly adjust the restriction of the food passageway.

144. An apparatus according to claim 1 or 8, wherein the adjustment means is powered.

145. An apparatus according to claim 1 or 8, wherein the operation device is powered.

146. An apparatus according to claim 1 or 8, wherein the adjustment means is operable in a non-invasive manner.

147. An apparatus according to claim 1 or 8, wherein the operation device is operable in a non-invasive manner.

## Patentansprüche

1. Vorrichtung zum Behandeln von Sodbrennen und Refluxerkrankungen, die ein einstellbares Begrenzungsmittel (2) umfasst, das beschaffen ist, um sich mit dem Magen nah beim Herz oder der Speiseröhre eines Patienten in Eingriff zu befinden, um einen eingeschränkten Nahrungsdurchgang in dem Magen oder der Speiseröhre zu bilden, das implantierbare Einstellmittel (5) zum postoperativen Einstellen der Begrenzungsmittel (2), um den Nahrungsdurchgang zu vergrößern oder einzuschränken, wenn die Begrenzungsmittel in den Patienten implantiert sind, eine implantierbare Hydraulikbetätigungsvorrichtung (6, 8; 10; 12; 10, 14; 76-86) zum Betätigen der Einstellmittel und einen Behälter (4; 10; 16), der in den Patienten implantierbar ist und ein Hydraulikfluid enthält, umfasst, wobei die Einstellmittel einen ausdehnbaren Hohlraum (5) in dem Begrenzungselement (2) umfassen, der Nahrungsdurchgang bei der Ausdehnung des Hohlraums eingeschränkt und bei der Kontraktion des Hohlraums vergrößert wird und die Hydraulikbetätigungsvorrichtung (6, 8; 10; 12; 10, 14; 76-86) beschaffen ist, um das Hydraulikfluid aus dem Behälter (4; 10; 16) zu verteilen, um den Hohlraum auszudehnen, und um das Hydraulikfluid aus dem Hohlraum in den Behälter zu verteilen, um den Hohlraum zusammenzuziehen, wobei der Behälter (4; 10; 16) eine vorgegebene Menge des Hydraulikfluids enthält, und wobei der Behälter (10) eine Kammer (42; 64) für die vorgegebene Menge des Fluids definiert und die Betätigungsvorrichtung beschaffen ist, um die Größe der Kammer zu ändern, und wobei die Betätigungsvorrichtung beschaffen ist, um die Größe der Kammer in Reaktion auf den Druck in dem Behälter zu ändern.

2. Vorrichtung nach Anspruch 1, wobei die Betätigungsvorrichtung einen ersten und einen zweiten Wandabschnitt (48; 66) des Behälters (10) umfasst und beschaffen ist, um eine relative Verschiebung zwischen dem ersten und dem zweiten Wandabschnitt des Behälters zu schaffen, um das Volumen der Kammer (42; 64) zu ändern.

3. Vorrichtung nach Anspruch 1, wobei die Betätigungsvorrichtung eine druckgesteuerte Hydraulikbetätigungsvorrichtung umfasst.

4. Vorrichtung nach Anspruch 2, wobei der erste und der zweite Wandabschnitt des Behälters (10) durch Magnetmittel, Hydraulikmittel oder elektrische Steuermittel oder eine Kombination aus diesen in Bezug aufeinander verschiebbar sind.

5. Vorrichtung nach Anspruch 2, wobei die Betätigungsvorrichtung beschaffen ist, um das Fluid in Reaktion auf eine vorgegebene erste Verschiebung des ersten Wandabschnitts (48; 66) des Behälters bezüglich des zweiten Wandabschnitts des Behälters aus dem Behälter (10) zu dem Hohlraum (5) der Begrenzungsvorrichtung (2) zu verteilen und in Reaktion auf eine vorgegebene zweite Verschiebung des ersten Wandabschnitts bezüglich des zweiten Wandabschnitts aus dem Hohlraum zu dem Behälter zu verteilen.

6. Vorrichtung nach einem der Ansprüche 1-5, wobei die Betätigungsvorrichtung Servomittel umfasst.

7. Vorrichtung nach einem der Ansprüche 2, 4 oder 5, wobei die Betätigungsvorrichtung einen gegensinnigen Servomechanismus umfasst.

8. Vorrichtung nach Anspruch 1, wobei die Betätigungsvorrichtung Servomittel umfasst, die betriebstechnisch mit der Einstellvorrichtung verbunden sind.

9. Vorrichtung nach Anspruch 1 oder 8, wobei die Betätigungsvorrichtung mit Energie versorgt wird.

10. Vorrichtung nach Anspruch 1 oder 8, wobei die Betätigungsvorrichtung manuell betätigt ist.

11. Vorrichtung nach Anspruch 8 oder 9, wobei die Servomittel einen Motor, vorzugsweise einen Elektromotor umfassen.

12. Vorrichtung nach Anspruch 11, wobei der Motor umkehrbar ist.

13. Vorrichtung nach Anspruch 11 oder 12, die ferner ein Getriebe umfasst, das zwischen den Motor und die Einstellvorrichtung gekoppelt ist.

14. Vorrichtung nach einem der Ansprüche 8-10, die ferner einen implantierbaren Behälter (4; 10; 16) umfasst, der eine Kammer für das Hydraulikfluid definiert, wobei die Betätigungsvorrichtung beschaffen ist, um die Einstellvorrichtung (5) unter Verwendung des Hydraulikfluids des Behälters zu betätigen.

15. Vorrichtung nach Anspruch 14, wobei der Behälter (4; 10; 16) eine vorgegebene Menge des Hydraulikfluids enthält.

16. Vorrichtung nach einem der Ansprüche 8-10 oder 14-15, wobei die Servomittel einen gegensinnigen Servomechanismus umfassen.

17. Vorrichtung nach den Ansprüchen 14 und 16, wobei der Behälter (204) einen ersten und einen zweiten Wandabschnitt umfasst und der gegensinnige Servomechanismus beschaffen ist, um eine relative Verschiebung zwischen dem ersten und dem zweiten Wandabschnitt des Behälters zu schaffen, um das Volumen des Behälters zu ändern.

18. Vorrichtung nach einem der Ansprüche 8-17, wobei die Servomittel druckgesteuerte Servomittel umfassen und
ferner ein Alarmsystem umfassen, das beschaffen ist, um in Reaktion auf das Verstreichen einer vorgegebenen Zeitdauer, während der der Druck, der die Servomittel steuert, einen vorgegebenen hohen Wert übersteigt, ein Alarmsignal zu erzeugen.

19. Vorrichtung nach Anspruch 16, wobei der gegensinnige Servomechanismus Magnetmittel, Elektromittel oder Mittel für die manuelle Betätigung oder eine Kombination daraus umfasst.

20. Vorrichtung nach Anspruch 17, wobei der gegensinnige Servomechanismus Hydraulikmittel (10, 14, 76-86) umfasst.

21. Vorrichtung nach Anspruch 20, wobei der gegensinnige Servomechanismus ferner einen Servobehälter umfasst, der eine Kammer definiert, die ein Servofluid enthält, und die Betätigungsvorrichtung einen ersten und einen zweiten Wandabschnitt des Servobehälters umfasst, die in Bezug aufeinander verschiebbar sind, um das Volumen der Kammer des Servobehälters zu ändern.

22. Vorrichtung nach Anspruch 21, wobei der erste und der zweite Wandabschnitt des Servobehälters durch Magnetmittel, Hydraulikmittel oder elektrische Steuermittel in Bezug aufeinander verschiebbar sind.

23. Vorrichtung nach Anspruch 20, wobei der gegensinnige Servomechanismus einen Servobehälter (14) und einen Fluidzufuhrbehälter (10) umfasst, die in einem geschlossenen System verbunden sind und eine weitere vorgegebene Menge des Fluids enthalten.

24. Vorrichtung nach Anspruch 23, wobei der Fluidzufuhrbehälter (10) eine Kammer für die weitere vorgegebene Menge des Fluids definiert und die Hydraulikbetätigungsvorrichtung beschaffen ist, um das Volumen der Kammer zu ändern und **dadurch** die Menge des Fluids im Servobehälter (14) zu steuern.

25. Vorrichtung nach Anspruch 24, wobei der Fluidzufuhrbehälter (10) einen ersten und einen zweiten Wandabschnitt umfasst, die in Bezug aufeinander verschiebbar sind, um das Volumen der Kammer des Fluidzufuhrbehälters zu ändern.

26. Vorrichtung nach Anspruch 25, wobei der Fluidzufuhrbehälter (10) in Reaktion auf eine vorgegebene erste Verschiebung des ersten Wandabschnitts des Fluidzufuhrbehälters bezüglich des zweiten Wandabschnitts des Fluidzufuhrbehälters die Menge des Fluids in dem Servobehälter (14) vergrößert und in Reaktion auf eine vorgegebene zweite Verschiebung des ersten Wandabschnitts des Fluidzufuhrbehälters bezüglich des zweiten Wandabschnitts des Fluidzufuhrbehälters die Menge des Fluids im Servobehälter verringert.

27. Vorrichtung nach Anspruch 1, die ferner eine Injektionsöffnung umfasst, die subkutan in den Patienten implantierbar ist und mit der Kammer in Fluidkommunikation steht.

28. Vorrichtung nach Anspruch 27, wobei die Injektionsöffnung in den Behälter integriert ist.

29. Vorrichtung nach Anspruch 1 oder 8, wobei die Begrenzungsmittel ein längliches Begrenzungselement und Formungsmittel zum Formen des Begrenzungselements in wenigstens eine im Wesentlichen geschlossene Schleife um die Speiseröhre oder den Magen umfassen, wobei die Schleife eine Begrenzungsöffnung definiert, wodurch die Einstellmittel beschaffen sind, um das Begrenzungselement in der Schleife einzustellen, um die Größe der Begrenzungsöffnung zu ändern.

30. Vorrichtung nach Anspruch 29, wobei die Formungsmittel das Begrenzungselement (2) in eine Schleife formen, die eine vorgegebene Größe oder eine Größe, die aus mehreren vorgegebenen Größen ausgewählt ist, besitzt.

31. Vorrichtung nach Anspruch 29, wobei die Einstellvorrichtung beschaffen ist, um die Größe der Begrenzungsöffnung so zu ändern, dass die äußere Umfangsbeschränkungsfläche des Begrenzungselements geändert wird.

32. Vorrichtung nach Anspruch 29, wobei die Einstellvorrichtung beschaffen ist, um die Größe der Begrenzungsmittel so zu ändern, dass die äußere Umfangsbeschränkungsfläche des Begrenzungselements unverändert ist.

33. Vorrichtung nach Anspruch 29, wobei das Begrenzungselement nicht aufblasbar ist und die Einstellvorrichtung beschaffen ist, um das Begrenzungselement in der Schleife einzustellen.

34. Vorrichtung nach Anspruch 22, wobei die Einstellvorrichtung das Begrenzungselement mechanisch einstellt.

35. Vorrichtung nach Anspruch 33, wobei die Einstellvorrichtung das nicht aufblasbare Begrenzungselement hydraulisch einstellt.

36. Vorrichtung nach Anspruch 34 oder 35, wobei das längliche Begrenzungselement (204) flexibel ist und die Einstellmittel (210) beschaffen sind, um einen ersten Abschnitt (204A) des flexiblen Begrenzungselements von einem zweiten Abschnitt (204B) des flexiblen Begrenzungselements, der dem ersten Abschnitt in der Schleife gegenüberliegt, zu ziehen, um die Speiseröhre (206) oder den Magen zwischen den gegenüberliegenden Längen des länglichen Begrenzungselements zusammenzudrücken, um den Durchgang einzuschränken, und um die Speiseröhre oder den Magen von dem flexiblen Begrenzungselement freizugeben, um den Durchgang zu vergrößern.

37. Vorrichtung nach einem der vorhergehenden Ansprüche, die ferner eine drahtlose Fernsteuerung (44, 126, 132-144) zum nichtinvasiven Steuern der Hydraulikbetätigungsvorrichtung (6, 8; 10; 12; 10, 14; 76-86) umfasst.

38. Vorrichtung nach Anspruch 37, wobei die Fernsteuerung eine externe drahtlose handgehaltene Fernsteuereinheit umfasst, die durch den Patienten manuell betätigbar ist, um die Begrenzungsvorrichtung zu steuern, um die Begrenzung des Nahrungsdurchganges zu ändern.

39. Vorrichtung nach Anspruch 37, wobei die Fernsteuerung (44, 126, 132-144) einen externen Signalsender (132, 136), Empfänger oder Sender/Empfänger und einen Signalempfänger (134, 138), Sender oder Sender/Empfänger, die in den Patienten implantierbar sind, umfasst.

40. Vorrichtung nach Anspruch 39, wobei der Signalempfänger (134, 138) und/oder der Sender/Empfänger eine Steuereinheit (138) umfasst, die beschaffen ist, um die Betätigungsvorrichtung (6, 8; 10; 12; 10, 14; 76-86) in Reaktion auf ein vom Signalsender (132, 136) empfangenes Steuersignal zu steuern.

41. Vorrichtung nach Anspruch 3, die ferner ein Alarmsystem umfasst, das beschaffen ist, um in Reaktion auf das Verstreichen einer vorgegebenen Zeitdauer, während der der Druck, der die Hydraulikbetätigungsvorrichtung steuert, einen vorgegebenen hohen Wert übersteigt, ein Alarmsignal zu erzeugen.

42. Vorrichtung nach Anspruch 40, die ferner eine implantierbare Energiequelleneinheit (136) umfasst, um den energieverbrauchenden implantierbaren Komponenten der Vorrichtung Energie bereitzustellen.

43. Vorrichtung nach Anspruch 1 oder 8 oder 42, wobei die Betätigungsvorrichtung einen Motor (44) zum Betätigen der Einstellvorrichtung umfasst.

44. Vorrichtung nach den Ansprüchen 42 und 43, wobei die Steuereinheit (138) beschaffen ist, um den Motor (44) in Reaktion auf ein von dem Signalsender (132, 136) empfangenes Steuersignal mit der von der Energiequelleneinheit (136) bereitgestellten Energie mit Energie zu versorgen.

45. Vorrichtung nach Anspruch 37, wobei die Fernsteuerung (44, 126, 132-144) Mittel zur drahtlosen Energieübertragung zum Übertragen von Energie von außerhalb des Körpers des Patienten zu den energieverbrauchenden implantierbaren Komponenten der Vorrichtung umfasst.

46. Vorrichtung nach Anspruch 42 und 45, wobei die Energieübertragungsmittel eine implantierbare Energiequelleneinheit (126) umfassen, die beschaffen ist, um die Energie von dem Steuersignal, wie es zu dem Signalempfänger (134, 138) übertragen wird, in elektrische Energie umzusetzen.

47. Vorrichtung nach Anspruch 45, wobei die Betätigungsvorrichtung (6; 8; 10; 12; 10, 14; 76-86) einen Motor (44) umfasst und die Mittel zur drahtlosen Energieübertragung beschaffen sind, um den Motor mit der übertragenen Energie direkt mit Energie zu versorgen.

48. Vorrichtung nach Anspruch 46 oder 47, wobei die durch die Mittel zur drahtlosen Energieübertragung übertragene Energie ein Signal umfasst.

49. Vorrichtung nach Anspruch 48, wobei das Signal ein Wellensignal umfasst.

50. Vorrichtung nach Anspruch 46 oder 47, wobei die durch die Mittel zur drahtlosen Energieübertragung übertragene Energie ein elektrisches Feld oder ein magnetisches Feld oder eine Kombination daraus umfasst.

51. Vorrichtung nach Anspruch 48, wobei das Signal analog oder digital oder eine Kombination daraus ist.

52. Vorrichtung nach Anspruch 39 oder 40, wobei der Signalsender (132, 136) und der Signalempfänger (134, 138) beschaffen sind, um ein analoges oder ein digitales Signal oder eine Kombination daraus zu senden und zu empfangen.

53. Vorrichtung nach Anspruch 51 oder 52, wobei das Signal analoge oder digitale Impulse umfasst.

54. Vorrichtung nach einem der Ansprüche 51-53, wobei das analoge oder das digitale Signal ein magnetisches Feld oder ein elektrisches Feld oder eine Kombination daraus umfasst.

55. Vorrichtung nach Anspruch 39 oder 40, wobei der Signalsender (132, 136) und der Signalempfänger (134, 138) beschaffen sind, um ein Wellensignal zu senden und zu empfangen.

56. Vorrichtung nach Anspruch 48 oder 55, wobei das Wellensignal ein elektromagnetisches Wellensignal, ein Schallwellensignal oder ein Trägerwellensignal für ein Fernsteuersignal oder eine Kombination daraus umfasst.

57. Vorrichtung nach Anspruch 56, wobei das Trägersignal frequenz-, amplituden- oder frequenz- und amplitudenmoduliert ist

58. Vorrichtung nach Anspruch 48 oder 51, wobei die Energieübertragungsmittel die Energie von dem Signal in einen Gleichstrom, einen pulsierenden Gleichstrom oder einen Wechselstrom oder eine Kombination daraus umsetzen.

59. Vorrichtung nach Anspruch 37, wobei die Fernsteuerung (44, 126, 132-144) Informationen, die mit wichtigen Parametern der Vorrichtung aus dem Inneren des Körpers des Patienten in Beziehung stehen, erhalten kann und der Einstellvorrichtung (5) in Reaktion auf die erhaltenen Informationen befehlen kann, die Begrenzungsvorrichtung (2) einzustellen.

60. Vorrichtung nach Anspruch 37, wobei die Fernsteuerung Informationen, die mit dem Nahrungsdurchgang in dem Magen oder der Speiseröhre in Beziehung stehen, empfangen kann und den Einstellmitteln in Reaktion auf die erhaltenen Informationen befehlen kann, die Begrenzungsmittel einzustellen.

61. Vorrichtung nach einem der Ansprüche 1 oder 8, die ferner eine implantierbare Energiequelle umfasst, um die Betätigungsvorrichtung mit Energie zu versorgen und/oder um den anderen energieverbrauchenden Komponenten der Vorrichtung Energie zuzuführen, wobei die Energie von der Energiequelle von außerhalb des Körpers des Patienten freigebbar ist.

62. Vorrichtung nach einem der Ansprüche 1 oder 8, die ferner eine Energieübertragungsvorrichtung für die drahtlose Übertragung von Energie umfasst.

63. Vorrichtung nach Anspruch 61 und 62, wobei die Energieübertragungsvorrichtung die Energie einer ersten Form überträgt, und die ferner eine Energieumsetzungsvorrichtung umfasst, die in den Patienten implantierbar ist, um die Energie der ersten Form in die Energie einer zweiten Form umzusetzen, um an die Energiequelle und/oder die anderen implantierbaren energieverbrauchenden Teile der Vorrichtung geliefert zu werden.

64. Vorrichtung nach Anspruch 63, wobei die Energie der zweiten Form anders als die Energie der ersten Form ist.

65. Vorrichtung nach Anspruch 63, wobei die Energieübertragungsvorrichtung von der Energieumsetzungsvorrichtung verschieden arbeitet.

66. Vorrichtung nach Anspruch 62, die ferner einen implantierbaren Motor oder eine implantierbare Pumpe zum Betätigen der Einstellvorrichtung umfasst, wobei die Energieübertragungsvorrichtung beschaffen ist, um drahtlose Energie in der Form eines Magnetfeldes oder elektromagnetischer Wellen oder eines Feldes für die direkte Versorgung des Motors oder der Pumpe mit Energie zu übertragen, wie die drahtlose Energie übertragen wird.

67. Vorrichtung nach Anspruch 40 und 62, wobei die Energieübertragungsvorrichtung die Energie durch wenigstens ein Signal überträgt, das von dem Steuersignal getrennt ist.

68. Vorrichtung nach Anspruch 63, die ferner einen eingebauten Stabilisator zum Stabilisieren der Energie der ersten oder der zweiten Form umfasst.

69. Vorrichtung nach Anspruch 68, wobei die Energie der zweiten Form elektrischen Strom umfasst und der Stabilisator wenigstens einen Kondensator umfasst.

70. Vorrichtung nach einem der Ansprüche 61, 63-65, wobei die Energiequelle eine Batterie, einen Akkumulator, einen Kondensator oder eine Kombination daraus umfasst.

71. Vorrichtung nach einem der Ansprüche 1 oder 8, die ferner eine Steuervorrichtung umfasst, die beschaffen ist, um drahtlose Energie zu erzeugen, um die Betätigungsvorrichtung direkt mit Energie zu versorgen und/oder um den anderen energieverbrauchenden Komponenten der Vorrichtung Energie zuzuführen.

72. Vorrichtung nach einem der Ansprüche 1 oder 8, die ferner eine implantierbare Energieumsetzungsvorrichtung zum Umsetzen der drahtlosen Energie direkt oder indirekt in von der drahtlosen Energie verschiedene Energie für den Betrieb der Begrenzungsvorrichtung umfasst.

73. Vorrichtung nach Anspruch 71 oder 72, wobei die drahtlose Energie ein Wellensignal umfasst, das ein Schallwellensignal, ein Ultraschallwellensignal, ein Signal elektromagnetischer Wellen, ein Infrarotlichtsignal, ein Signal sichtbaren Lichts, ein Ultraviolettlichtsignal, ein Laserlichtsignal, ein Mikrowellensignal oder ein Funkwellensignal enthält.

74. Vorrichtung nach einem der Ansprüche 1 oder 8, die ferner Energieübertragungsmittel (22, 326, 332-344) für die drahtlose Übertragung von Energie von außerhalb des Körpers des Patienten zu der Betätigungsvorrichtung oder der Einstellvorrichtung und/oder den anderen energieverbrauchenden implantierbaren Komponenten der Vorrichtung umfasst.

75. Vorrichtung nach Anspruch 71, wobei die Steuervorrichtung beschaffen ist, um die drahtlose Energie in der Form eines Zuges von Energieimpulsen zu erzeugen.

76. Vorrichtung nach Anspruch 45 oder 74, wobei die Energieübertragungsmittel beschaffen sind, um die Energie in der Form eines Zuges von Energieimpulsen für die direkte Verwendung im Zusammenhang mit dem Zuführen von Energie zu den energieverbrauchenden Komponenten der Vorrichtung intermittierend zu übertragen.

77. Vorrichtung nach Anspruch 71 und 63, wobei die Steuervorrichtung beschaffen ist, um die Energieumsetzungsvorrichtung zu steuern, um die Energie der zweiten Form in einem Zug von Energieimpulsen für die direkte Verwendung im Zusammenhang mit dem Betrieb der Einstellvorrichtung zu erzeugen.

78. Vorrichtung nach Anspruch 76 oder 77, wobei die Energieübertragungsvorrichtung beschaffen ist, um elektrische Energie zu übertragen, und ferner einen implantierbaren Kondensator zum Erzeugen des Zuges von Energieimpulsen umfasst.

79. Vorrichtung nach Anspruch 78 oder 69, 70, wobei der Kondensator eine Kapazität von kleiner als 0,1 µF besitzt.

80. Vorrichtung nach Anspruch 74, die ferner einen implantierbaren Motor (22) oder eine implantierbare Pumpe zum Betätigen der Einstellvorrichtung (12; 52; 66; 90, 92; 104; 110) umfasst, wobei die Energieübertragungsmittel beschaffen sind, um den Motor oder die Pumpe mit der übertragenen Energie direkt mit Energie zu versorgen.

81. Vorrichtung nach Anspruch 66 oder 80, wobei die Pumpe kein Tauchkolbentyp der Pumpe ist.

82. Vorrichtung nach einem der Ansprüche 38-81, die ferner eine drahtlose Fernsteuerung (44, 126, 132-144) zum nichtinvasiven Steuern der Betätigungsvorrichtung (6, 8; 10; 12; 10, 14; 76-86) umfasst.

83. Vorrichtung nach einem der vorhergehenden Ansprüche, wobei die Betätigungsvorrichtung elektrisch mit Energie versorgt wird.

84. Vorrichtung nach einem der Ansprüche 1, 8 oder 66, wobei die Betätigungsvorrichtung durch statische permanente magnetische Energie nicht mit Energie versorgt werden kann.

85. Vorrichtung nach einem der Ansprüche 1 oder 8, wobei die Betätigungsvorrichtung beschaffen ist, um die Einstellvorrichtung nichtinvasiv zu betätigen.

86. Vorrichtung nach einem der Ansprüche 1 oder 8, wobei die Begrenzungsmittel betreibbar sind, um die Begrenzung des Nahrungsmitteldurchganges stufenlos einzustellen.

87. Vorrichtung nach Anspruch 1 oder 8, wobei die Betätigungsvorrichtung eine Hydraulikbetätigungsvorrichtung umfasst, die ein Hydraulikfluid verwendet, dessen Viskosität sich ändert, wenn das Hydraulikfluid einer Energie ausgesetzt ist, die von thermischer Energie verschieden ist.

88. Vorrichtung nach Anspruch 83, wobei sich die Viskosität des Hydraulikfluids ändert, wenn das Fluid elektrischer Energie ausgesetzt ist.

89. Vorrichtung nach Anspruch 1 oder 8, die ferner eine Einstellvorrichtung zum Einstellen der Begrenzungsvorrichtung umfasst, um die Begrenzung des Nahrungsaufnahme-Durchgangs zu ändern, wobei die Einstellvorrichtung beschaffen ist, um die Begrenzungsvorrichtung mechanisch einzustellen, oder beschaffen ist, um die Begrenzungsvorrichtung unter Verwendung von Hydraulikmitteln, die kein Hydraulikfluid der Art aufweisen, das eine Viskosität besitzt, die sich beträchtlich vergrößert, wenn es Wärme oder einem Magnetfeld ausgesetzt ist, hydraulisch einzustellen.

90. Vorrichtung nach Anspruch 1 oder 8, die ferner eine Steuervorrichtung zum Steuern der Begrenzungsvorrichtung umfasst.

91. Vorrichtung nach Anspruch 90, wobei die Steuervorrichtung eine interne Steuereinheit umfasst, die in den Patienten implantierbar ist, um die Begrenzungsvorrichtung zu steuern.

92. Vorrichtung nach Anspruch 91, wobei die interne Steuereinheit programmierbar ist.

93. Vorrichtung nach Anspruch 92, wobei die Steuervorrichtung eine externe Steuereinheit außerhalb des Körpers des Patienten umfasst, wobei die implantierbare interne Steuereinheit durch die externe Steuereinheit programmierbar ist.

94. Vorrichtung nach Anspruch 90, wobei die Steuervorrichtung eine externe Steuereinheit außerhalb des Körpers des Patienten umfasst, um die Begrenzungsvorrichtung drahtlos zu steuern.

95. Vorrichtung nach Anspruch 94, wobei die externe Steuereinheit programmierbar ist.

96. Vorrichtung nach einem der vorhergehenden Ansprüche, die ferner wenigstens einen implantierbaren Sensor umfasst, um wenigstens einen physischen Parameter des Patienten abzutasten.

97. Vorrichtung nach Anspruch 96, wobei der Sensor beschaffen ist, um die horizontale Position des Patienten als den physischen Parameter direkt oder indirekt abzutasten.

98. Vorrichtung nach Anspruch 96, wobei der Sensor einen Drucksensor umfasst, um den Druck gegen die Begrenzungsvorrichtung oder einen Teil des menschlichen Körpers als den physischen Parameter direkt oder indirekt abzutasten.

99. Vorrichtung nach Anspruch 98, wobei die Begrenzungsmittel beschaffen sind, um den Nahrungsdurchgang in dem Magen oder in der Speiseröhre in Reaktion darauf, dass der Drucksensor einen vorgegebenen Druck abtastet, zu vergrößern.

100. Vorrichtung nach einem der Ansprüche 96-99, die ferner eine Steuervorrichtung zum Steuern der Begrenzungsvorrichtung in Reaktion auf die Signale von dem Sensor umfasst.

101. Vorrichtung nach Anspruch 100, wobei die Steuervorrichtung eine interne Steuereinheit umfasst, die in den Patienten implantierbar ist und die die Begrenzungsvorrichtung in Reaktion auf die Signale von dem Sensor direkt steuert.

102. Vorrichtung nach Anspruch 101, wobei die Steuervorrichtung eine externe Steuereinheit außerhalb des Körpers des Patienten umfasst, um die Begrenzungsvorrichtung in Reaktion auf die Signale von dem Sensor zu steuern.

103. Vorrichtung nach Anspruch 101, wobei die Steuervorrichtung eine externe Steuereinheit außerhalb des Körpers des Patienten umfasst, um die Begrenzungsvorrichtung in Reaktion auf die Informationen von dem Sensor manuell zu steuern.

104. Vorrichtung nach Anspruch 90, die ferner eine implantierbare Energiequelle umfasst, wobei die Steuervorrichtung von außerhalb des Körpers des Patienten betätigbar ist, um die Energiequelle zu steuern, um die Energie die für die Verwendung im Zusammenhang mit dem Betrieb der Prothese freizugeben, wenn die Prothese implantiert ist.

105. Vorrichtung nach Anspruch 104, wobei die Energiequelle außerhalb des Körpers des Patienten vorgesehen ist und die Steuervorrichtung beschaffen ist, um die externe Energiequelle zu steuern, um drahtlose Energie für die Verwendung im Zusammenhang mit dem Betrieb der Prothese freizugeben.

106. Vorrichtung nach Anspruch 104, wobei die Steuervorrichtung die Energiequelle steuert, um magnetische Energie, nichtmagnetische Energie, elektromagnetische Energie, nichtelektromagnetische Energie, kinetische Energie, nichtkinetische Energie, Schallenergie, Nichtschallenergie, thermische Energie oder nichtthermische Energie freizugeben.

107. Vorrichtung nach Anspruch 104, wobei die Steuervorrichtung die Energiequelle steuert, um die Energie während einer vorgegebenen Zeitdauer freizugeben.

108. Vorrichtung nach Anspruch 104 oder 104 und 75, wobei die Steuervorrichtung die Energiequelle steuert, um die Energie in einer vorgegebenen Anzahl von Energieimpulsen freizugeben.

109. Vorrichtung nach Anspruch 104, wobei die Steuervorrichtung beschaffen ist, um die Energiequelle zu steuern, um die Energie auf eine nichtinvasive Art freizugeben.

110. Vorrichtung nach einem der Ansprüche 1 oder 8, die ferner implantierbare elektrische Komponenten umfasst, die wenigstens einen Spannungspegelschutz umfassen.

111. Vorrichtung nach einem der Ansprüche 1 oder 8, die ferner implantierbare elektrische Komponenten umfasst, die einen einzigen Spannungspegelschutz umfassen.

112. Vorrichtung nach Anspruch 110 oder 111, wobei die elektrischen Komponenten keinen Stromdetektor und/oder keinen Ladungspegeldetektor aufweisen.

113. Vorrichtung nach einem der Ansprüche 61, 78, 63, 69 oder 70 und 110-112, die ferner einen implantierbaren Kondensator oder Akkumulator umfasst, wobei die Ladung oder die Entladung des Kondensators oder des Akkumulators unter Verwendung des Spannungspegelschutzes gesteuert wird.

114. Vorrichtung nach Anspruch 40, 46 oder 67, wobei das Steuersignal ein Wellensignal umfasst, das ein Schallwellensignal, ein Ultraschallwellensignal, ein Signal elektromagnetischer Wellen, ein Infrarotlichtsignal, ein Signal sichtbaren Lichts, ein Ultraviolettlichtsignal, ein Laserlichtsignal, ein Mikrowellensignal oder ein Funkwellensignal enthält.

115. Vorrichtung nach einem der vorhergehenden Ansprüche, die ferner einen Schalter umfasst, der in den Patienten implantierbar ist, um den Betrieb der Begrenzungsvorrichtung direkt oder indirekt zu schalten.

116. Vorrichtung nach Anspruch 115 und 62, die ferner eine Energiequelle umfasst, die in den Patienten implantierbar ist, um die Energie für den Betrieb der Begrenzungsvorrichtung zu liefern, wobei der Schalter durch die Energie betätigt wird, die von der Energieübertragungsvorrichtung geliefert wird, um aus einer Aus-Betriebsart, in der sich die Energiequelle nicht in Gebrauch befindet, in eine Ein-Betriebsart, in der die Energiequelle die Energie für den Betrieb der Begrenzungsvorrichtung liefert, zu schalten.

117. Vorrichtung nach Anspruch 115 und 62, die ferner eine Energiequelle, die in den Patienten implantierbar ist, um die Energie für den Betrieb der Begrenzungsvorrichtung zu liefern, und eine Fernsteuerung zum Steuern der Zufuhr der Energie der implantierbaren Energiequelle umfasst, wobei der Schalter durch die Energie betätigt wird, die von der Energieübertragungsvorrichtung geliefert wird, um aus einer Aus-Betriebsart, in der verhindert wird, dass die Fernsteuerung die Energiequelle steuert, und in der sich die Energiequelle nicht in Gebrauch befindet, in eine Bereitschaftsbetriebsart, in der es der Fernsteuerung erlaubt ist, die Energiequelle zu steuern, um die Energie für den Betrieb der Begrenzungsvorrichtung zu liefern, zu schalten.

118. Vorrichtung nach Anspruch 115 und 63, die ferner eine Energiequelle umfasst, die in den Patienten implantierbar ist, um die Energie für den Betrieb der Begrenzungsvorrichtung zu liefern, wobei der Schalter durch die von der Energieumsetzungsvorrichtung gelieferte Energie betätigt wird, um aus einer Aus-Betriebsart, in der sich die Energiequelle nicht in Gebrauch befindet, in eine Ein-Betriebsart, in der die Energiequelle die Energie für den Betrieb der Begrenzungsvorrichtung liefert, zu schalten.

119. Vorrichtung nach Anspruch 115 und 63, die ferner eine Energiequelle, die in den Patienten implantierbar ist, um die Energie für den Betrieb der Begrenzungsvorrichtung zu liefern, und eine Fernsteuerung zum Steuern der Zufuhr der Energie der implantierbaren Energiequelle umfasst, wobei der Schalter durch die von der Energieumsetzungsvorrichtung gelieferte Energie betätigt wird, um aus einer Aus-Betriebsart, in der verhindert wird, dass die Fernsteuerung die Energiequelle steuert, und in der sich die Energiequelle nicht in Gebrauch befindet, in eine Bereitschaftsbetriebsart, in der es der Fernsteuerung erlaubt ist, die Energiequelle zu steuern, um die Energie für den Betrieb der Begrenzungsvorrichtung zu liefern, zu schalten.

120. Vorrichtung nach einem der Ansprüche 1 oder 8, wobei die Prothese betreibbar ist, um eine reversible Funktion auszuführen.

121. Vorrichtung nach Anspruch 42 oder 120, die ferner eine Umkehrvorrichtung umfasst, die in den Patienten implantierbar ist, um die durch die Prothese ausgeführte Funktion umzukehren.

122. Vorrichtung nach Anspruch 121, wobei die Steuervorrichtung die Umkehrvorrichtung steuert, um die durch die Prothese ausgeführte Funktion umzukehren.

123. Vorrichtung nach Anspruch 121, wobei die Umkehrvorrichtung Hydraulikmittel umfasst, die ein Ventil enthalten, um die Strömungsrichtung eines strömenden Fluids in den Hydraulikmitteln zu ändern.

124. Vorrichtung nach Anspruch 121, wobei die Umkehrvorrichtung eine mechanische Umkehrvorrichtung umfasst.

125. Vorrichtung nach Anspruch 121, wobei die Umkehrvorrichtung einen Schalter umfasst.

126. Vorrichtung nach Anspruch 125, wobei der Schalter der Umkehrvorrichtung durch die freigegebene Energie betätigbar ist.

127. Vorrichtung nach Anspruch 126, wobei die Steuervorrichtung den Betrieb des Schalters der Umkehrvorrichtung durch das Ändern der Polarität der an den Schalter gelieferten freigegebenen Energie steuert.

128. Vorrichtung nach Anspruch 121, wobei die Betätigungsvorrichtung einen Motor umfasst und die Umkehrvorrichtung den Motor umkehrt.

129. Vorrichtung nach einem der vorhergehenden Ansprüche, wobei die Begrenzungsvorrichtung in einem weichen oder gelartigen Material eingebettet ist.

130. Vorrichtung nach Anspruch 129, wobei die Begrenzungsvorrichtung in einem Siliconmaterial mit einer Härte, die kleiner als 20 Shore ist, eingebettet ist.

131. Vorrichtung nach Anspruch 63, wobei die Energieumsetzungsmittel oder -vorrichtung konstruiert sind, um subkutan oder im Bauch, im Brustkorb oder im Schädelbereich des Patienten implantiert zu werden.

132. Vorrichtung nach einem der vorhergehenden Ansprüche, wobei die Einstellvorrichtung beschaffen ist, um die Begrenzungsvorrichtung so einzustellen, dass die Begrenzungsvorrichtung eine vorgegebene Kontraktion des Nahrungsdurchgangs schafft, die für den Patienten zufriedenstellend ist.

133. Vorrichtung nach einem der Ansprüche 1 oder 8, wobei die Einstellvorrichtung beschaffen ist, um die Begrenzungsvorrichtung auf eine flusslose magnetische, nichtthermische oder die Viskosität nicht ändernde Art einzustellen.

134. Vorrichtung nach Anspruch 1, wobei die Einstellmittel einen ausdehnbaren Hohlraum in den Begrenzungsmitteln umfassen, der Nahrungsdurchgang bei der Ausdehnung des Hohlraums begrenzt und bei der Kontraktion des Hohlraums vergrößert wird und die Hydraulikbetätigungsmittel eine Injektionsöffnung umfassen, die subkutan in den Patienten implantierbar ist, um Fluid transkutan zu dem Hohlraum hinzufügen und um Fluid transkutan aus dem Hohlraum abzuziehen.

135. Vorrichtung nach Anspruch 1 oder 8, wobei die Einstellmittel Hydraulikbetätigungsmittel umfassen und die Hydraulikbetätigungsmittel eine Injektionsöffnung umfassen, die subkutan in den Patienten implantierbar ist, um Fluid transkutan zu den Hydraulikbetätigungsmitteln hinzufügen und um Fluid transkutan aus den Hydraulikbetätigungsmitteln abzuziehen.

136. Vorrichtung nach Anspruch 135, wobei die Injektionsöffnung nur für Eichzwecke verwendet wird und die Einstellvorrichtung ferner die Nahrungsbegrenzungsöffnung vergrößern kann, wenn Nahrung hindurchgehen sollte, wenn der Patient isst und Nahrung schluckt.

137. Vorrichtung nach Anspruch 136 und 8, wobei der Servomechanismus hydraulisch betätigt wird und wobei die Injektionsöffnung nur für Eichzwecke des Servomechanismus verwendet wird.

138. Vorrichtung nach Anspruch 2, wobei der erste und der zweite Wandabschnitt (66) des Behälters konstruiert sind, um durch ihre manuelle Betätigung in Bezug aufeinander verschiebbar zu sein.

139. Vorrichtung nach einem der vorhergehenden Ansprüche, die ferner wenigstens eine Haltevorrichtung umfasst, die in den Patienten implantierbar ist, um die Speiseröhre oder den Magen in einer Position zu halten, wo sich der linke und der rechte Schenkelmuskel befinden, um zu verhindern, dass sich der Bereich des Mageneingangs wegen des Zwerchfellmuskels kranial bewegt.

140. Vorrichtung nach Anspruch 139, wobei die Haltevorrichtung in Eingriff mit der Nahrungsbegrenzungsvorrichtung angeordnet ist.

141. Vorrichtung nach Anspruch 1 oder 8, wobei die Begrenzungsmittel betreibbar sind, um den Nahrungsdurchgang zu öffnen und zu schließen.

142. Vorrichtung nach Anspruch 141, wobei die Begrenzungsmittel betreibbar sind, um den Nahrungsdurchgang zu öffnen, wenn Nahrung hindurchgehen sollte und der Patient schluckt, und anderweitig den Nahrungsdurchgang zu schließen, um den Rückfluss von Säure aus dem Magen zu verhindern.

143. Vorrichtung nach Anspruch 1 oder 8, wobei die Begrenzungsmittel betreibbar sind, um die Begrenzung des Nahrungsdurchgangs stufenlos einzustellen.

144. Vorrichtung nach Anspruch 1 oder 8, wobei die Einstellmittel mit Energie versorgt werden.

145. Vorrichtung nach Anspruch 1 oder 8, wobei die Betätigungsvorrichtung mit Energie versorgt wird.

146. Vorrichtung nach Anspruch 1 oder 8, wobei die Einstellmittel auf eine nichtinvasive Art betreibbar sind.

147. Vorrichtung nach Anspruch 1 oder 8, wobei die Betätigungsvorrichtung auf eine nichtinvasive Art betreibbar ist.

## Revendications

1. Dispositif de traitement des pathologies de pyrosis et régurgitation, comprenant un moyen de restriction (2) réglable apte à venir au contact de l'estomac d'un patient, près du cardia ou de l'oesophage, pour former dans l'estomac ou l'oesophage un passage restreint pour la nourriture, comprenant un moyen de réglage (5) implantable pour un réglage postopératoire du moyen de restriction (2) afin d'agrandir ou de limiter le passage de la nourriture, quand le moyen de restriction est implanté dans le patient, un dispositif hydraulique de mise en fonctionnement (6, 8 ; 10 ; 12 ; 10, 14 ; 76-86) implantable pour faire marcher le moyen de réglage et un réservoir (4 ; 10 ; 16) implantable dans le patient et contenant un fluide hydraulique, dans lequel le moyen de réglage comprend une cavité (5) expansible dans l'élément de restriction (2), le passage de la nourriture étant limité quand la cavité se dilate et agrandi quand la cavité se contracte, et le dispositif hydraulique de mise en fonctionnement (6, 8 ; 10 ; 12 ; 10, 14 ; 76-86) est apte à distribuer du fluide hydraulique provenant du réservoir (4 ; 10 ; 16) pour dilater la cavité et à distribuer du fluide hydraulique provenant de la cavité vers le réservoir pour contracter la cavité, dans lequel le réservoir (4 ; 10 ; 16) contient une quantité prédéterminée de fluide hydraulique et dans lequel le réservoir (10) délimite une chambre (42 ; 64) pour ladite quantité prédéterminée de fluide et le dispositif de mise en fonctionnement est apte à modifier la taille de la chambre et dans lequel le dispositif de mise en fonctionnement est apte à modifier la taille de la chambre en réaction à la pression dans le réservoir.

2. Appareil selon la revendication 1, dans lequel le dispositif de mise en fonctionnement comprend une première et une seconde parties (48 ; 66) de cloison du réservoir (10) et est apte à assurer un déplacement relatif entre les première et seconde parties de cloison du réservoir afin de modifier le volume de la chambre (42 ; 64).

3. Appareil selon la revendication 1, dans lequel le dispositif de mise en fonctionnement comprend un dispositif de mise en fonctionnement hydraulique pressostatique.

4. Appareil selon la revendication 2, dans lequel les première et une seconde parties de cloison du réservoir (10) peuvent être déplacées l'une par rapport à l'autre par des moyens magnétiques, des moyens hydrauliques ou des moyens de commande électrique ou une combinaison de ceux-ci.

5. Appareil selon la revendication 2, dans lequel le dispositif de mise en fonctionnement est apte à distribuer du fluide du réservoir (10) vers la cavité (5) du dispositif de restriction (2) en réaction à un premier déplacement prédéterminé de la première partie (48 ; 66) de cloison du réservoir par rapport à la seconde partie de cloison du réservoir et à distribuer du fluide de la cavité vers le réservoir en réaction à un second déplacement prédéterminé de la première partie de cloison par rapport à la seconde partie de cloison.

6. Appareil selon l'une quelconque des revendications 1 à 5, dans lequel le dispositif de mise en fonctionnement comprend un moyen d'asservissement.

7. Appareil selon l'une quelconque des revendications 2, 4 et 5, dans lequel le dispositif de mise en fonctionnement comprend un servomécanisme inverse.

8. Appareil selon la revendication 1, dans lequel le dispositif de mise en fonctionnement comprend un moyen d'asservissement relié de façon fonctionnelle au dispositif de réglage.

9. Appareil selon la revendication 1 ou 8, dans lequel le dispositif de mise en fonctionnement est relié à une source d'énergie.

10. Appareil selon la revendication 1 ou 8, dans lequel le dispositif de mise en fonctionnement se manoeuvre manuellement.

11. Appareil selon la revendication 8 ou 9, dans lequel le moyen d'asservissement comprend un moteur, de préférence un moteur électrique.

12. Appareil selon la revendication 11, dans lequel le moteur est réversible.

13. Appareil selon la revendication 11 ou 12, comprenant en outre une pignonnerie assurant la liaison entre le moteur et le dispositif de réglage.

14. Appareil selon l'une quelconque des revendications 8 à 10, comprenant en outre un réservoir (4 ; 10 ; 16) implantable délimitant une chambre pour du fluide hydraulique, dans lequel le dispositif de mise en fonctionnement est apte à faire fonctionner dispositif de réglage (5) au moyen du fluide hydraulique du réservoir.

15. Appareil selon la revendication 14, dans lequel le réservoir (4 ; 10 ; 16) contient une quantité prédéterminée de fluide hydraulique.

16. Appareil selon l'une quelconque des revendications 8 à 10 et 14 et 15, dans lequel le moyen d'asservissement comprend un servomécanisme inverse.

17. Appareil selon les revendications 14 et 16, dans lequel le réservoir (204) comprend une première et une seconde parties de cloison et le servomécanisme inverse est apte à assurer un déplacement relatif entre les première et seconde parties de cloison du réservoir, afin de modifier le volume de ce dernier.

18. Appareil selon l'une quelconque des revendications 8 à 17, dans lequel le moyen d'asservissement comprend un servomécanisme pressostatique et qui comprend en outre une alarme apte à produire un signal d'alarme en réaction à l'écoulement d'une période prédéterminée pendant laquelle la pression commandant le moyen d'asservissement dépasse une valeur haute prédéterminée.

19. Appareil selon la revendication 16, dans lequel le servomécanisme inverse comprend des moyens magnétiques, des moyens électriques ou des moyens de manipulation manuelle ou une combinaison de ceux-ci.

20. Appareil selon la revendication 17, dans lequel le servomécanisme inverse comprend des moyens hydrauliques (10, 14, 76-86).

21. Appareil selon la revendication 20, dans lequel le servomécanisme inverse comprend un réservoir d'asservissement délimitant une chambre contenant du fluide d'asservissement et le dispositif de mise en fonctionnement comprend une première et une seconde parties de cloison du réservoir d'asservissement, qui peuvent être déplacées l'une par rapport à l'autre pour modifier le volume de la chambre du réservoir d'asservissement.

22. Appareil selon la revendication 21, dans lequel les première et seconde parties de cloison du réservoir d'asservissement peuvent être déplacées l'une par rapport à l'autre par des moyens magnétiques, des moyens hydrauliques ou des moyens de commande électrique.

23. Appareil selon la revendication 20, dans lequel le servomécanisme inverse comprend un réservoir d'asservissement (14) et un réservoir d'alimentation en fluide (10) raccordés dans un système fermé et contenant une quantité supplémentaire prédéterminée de fluide.

24. Appareil selon la revendication 23, dans lequel le réservoir d'alimentation en fluide (10) délimite une chambre pour la quantité supplémentaire prédéterminée de fluide et le dispositif de fonctionnement hydraulique est apte à modifier le volume de la chambre et par conséquent commander la quantité de fluide dans le réservoir d'asservissement (14).

25. Appareil selon la revendication 24, dans lequel le réservoir d'alimentation en fluide (10) comprend une première et une seconde parties de cloison, qui peuvent se déplacer l'une par rapport à l'autre pour modifier le volume de la chambre du réservoir d'alimentation en fluide.

26. Appareil selon la revendication 25, dans lequel le réservoir d'alimentation en fluide (10) augmente la quantité de fluide dans le réservoir d'asservissement (14) en réaction à un premier déplacement prédéterminé de la première partie de cloison du réservoir d'alimentation en fluide par rapport à la seconde partie de cloison du réservoir d'alimentation en fluide et diminue la quantité de fluide dans le réservoir d'asservissement en réaction à un second déplacement prédéterminé de la première partie de cloison du réservoir d'alimentation en fluide par rapport à la seconde partie de cloison du réservoir d'alimentation en fluide.

27. Appareil selon la revendication 1, comprenant en outre un orifice d'injection implantable dans le patient et en communication fluide avec la chambre.

28. Appareil selon la revendication 27, dans lequel l'orifice d'injection est intégré au réservoir.

29. Appareil selon la revendication 1 ou 8, dans lequel le moyen de restriction comprend un élément de restriction allongé et des moyens de formage pour façonner l'élément de restriction en au moins une boucle sensiblement fermée autour de l'oesophage ou de l'estomac, la boucle délimitant une ouverture de restriction, de sorte que le moyen de réglage est apte à régler l'élément de restriction dans la boucle pour modifier la taille de l'ouverture de restriction.

30. Appareil selon la revendication 29, dans lequel les moyens de formage façonnent l'élément de restriction (2) en une boucle ayant une taille prédéterminée ou une taille choisie parmi plusieurs tailles prédéterminées.

31. Appareil selon la revendication 29, dans lequel le dispositif de réglage est apte à modifier la taille de l'ouverture de restriction de telle sorte que la surface extérieure de confinement circonférentiel de l'élément de restriction soit modifiée.

32. Appareil selon la revendication 29, dans lequel le dispositif de réglage est apte à modifier la taille de l'ouverture de restriction de telle sorte que la surface extérieure de confinement circonférentiel de l'élément de restriction soit inchangée.

33. Appareil selon la revendication 29, dans lequel l'élément de restriction est non gonflable et le dispositif de réglage est apte à régler l'élément de restriction dans ladite boucle.

34. Appareil selon la revendication 22, dans lequel le dispositif de réglage ajuste mécaniquement l'élément de restriction.

35. Appareil selon la revendication 33, dans lequel le dispositif de réglage ajuste hydrauliquement l'élément de restriction non gonflable.

36. Appareil selon la revendication 34 ou 35, dans lequel l'élément de restriction (204) allongé est flexible et le moyen de réglage (210) est apte à tirer sur une première partie (204A) de l'élément de restriction flexible à partir d'une seconde partie (204B) de l'élément de restriction flexible située en face de la première partie dans la boucle pour presser l'oesophage (206) ou l'estomac entre les deux longueurs opposées de l'élément de restriction allongé flexible pour restreindre le passage ou à libérer l'oesophage ou l'estomac de l'élément de restriction flexible pour agrandir le passage.

37. Appareil selon l'une quelconque des revendications précédentes, comprenant en outre une télécommande sans fil (44, 126, 132-144) pour commander de façon non effractive le dispositif hydraulique de mise en fonctionnement (6, 8 ; 10 ; 12 ; 10, 14 ; 76-86).

38. Appareil selon la revendication 37, dans lequel la télécommande comprend une unité de télécommande portative sans fil externe que le patient peut faire marcher manuellement pour commander le dispositif de restriction et modifier la restriction du passage de la nourriture.

39. Appareil selon la revendication 37, dans lequel la télécommande (44, 126, 132-144) comprend un émetteur de signaux (132, 136) externe, un récepteur ou émetteur-récepteur et un récepteur de signaux (134, 138), émetteur ou émetteur-récepteur implantable dans le patient.

40. Appareil selon la revendication 39, dans lequel le récepteur de signaux (134, 138) et/ou émetteur-récepteur comprennent une unité de commande (138) apte à commander le dispositif de mise en fonctionnement (6, 8 ; 10 ; 12 ; 10, 14 ; 76-86) en réaction à un signal de commande reçu de l'émetteur de signaux (132, 136).

41. Appareil selon la revendication 3, comprenant en outre une alarme apte à produire un signal d'alarme en réaction à l'écoulement d'une période prédéterminée pendant laquelle la pression commandant le dispositif hydraulique de mise en fonctionnement dépasse une valeur haute prédéterminée.

42. Appareil selon la revendication 40, comprenant en outre une unité d'alimentation électrique (136) implantable pour fournir de l'énergie aux composants implantables énergivores de l'appareil.

43. Appareil selon la revendication 1 ou 8 ou 42, dans lequel le dispositif de mise en fonctionnement comprend un moteur électrique (44) pour faire fonctionner le dispositif de réglage.

44. Appareil selon les revendications 42 et 43, dans lequel l'unité de commande (138) est apte à faire marcher le moteur électrique (44) avec de l'énergie fournie par l'unité d'alimentation électrique (136) en réaction à un signal de commande reçu de l'émetteur de signaux (132, 136).

45. Appareil selon la revendication 37, dans lequel la télécommande (44, 126, 132-144) comprend un moyen de transfert d'énergie sans fil pour transférer de l'énergie de l'extérieur du corps du patient vers les composants implantables énergivores de l'appareil.

46. Appareil selon les revendications 42 et 45, dans lequel le moyen de transfert d'énergie comprend une unité d'alimentation électrique (126) implantable qui est apte à transformer en énergie électrique l'énergie provenant du signal de commande lorsqu'il est transmis au récepteur de signaux (134, 138).

47. Appareil selon la revendication 45, dans lequel le dispositif de mise en fonctionnement (6, 8 ; 10 ; 12 ; 10, 14 ; 76-86) comprend un moteur électrique (44) et le moyen de transfert d'énergie sans fil est apte à alimenter directement le moteur électrique avec de l'énergie transférée.

48. Appareil selon la revendication 46 ou 47, dans lequel l'énergie transférée par le moyen de transfert d'énergie sans fil comprend un signal.

49. Appareil selon la revendication 48, dans lequel ledit signal comprend un signal ondulatoire.

50. Appareil selon la revendication 46 ou 47, dans lequel l'énergie transférée par le moyen de transfert d'énergie sans fil comprend un champ électrique ou un champ magnétique ou une combinaison de ceux-ci.

51. Appareil selon la revendication 48, dans lequel le signal est analogique ou numérique ou une combinaison des deux.

52. Appareil selon la revendication 39 ou 40, dans lequel l'émetteur de signaux (132, 136) et le récepteur de signaux (134, 138) sont aptes à émettre et recevoir un signal analogique ou numérique ou une combinaison des deux.

53. Appareil selon la revendication 51 ou 52, dans lequel le signal comprend des impulsions analogiques ou numériques.

54. Appareil selon l'une quelconque des revendications 51 à 53, dans lequel le signal analogique ou numérique comprend un champ magnétique ou un champ électrique ou une combinaison de ceux-ci.

55. Appareil selon la revendication 39 ou 40, dans lequel l'émetteur de signaux (132, 136) et le récepteur de signaux (134, 138) sont aptes à émettre et recevoir un signal ondulatoire.

56. Appareil selon la revendication 48 ou 55, dans lequel le signal ondulatoire comprend un signal d'onde électromagnétique, un signal d'onde acoustique ou un signal d'onde porteuse pour un signal de télécommande ou une combinaison de ceux-ci.

57. Appareil selon la revendication 56, dans lequel le signal porteur est modulé en fréquence ou en amplitude ou en fréquence et amplitude.

58. Appareil selon la revendication 48 ou 51, dans lequel le moyen de transfert d'énergie transfère l'énergie provenant du signal dans un courant continu, continu pulsé ou alternatif ou une combinaison de ceux-ci.

59. Appareil selon la revendication 37, dans lequel la télécommande (44, 126, 132-144) est capable d'obtenir des informations concernant d'importants paramètres de l'appareil depuis l'intérieur du corps du patient et de commander le dispositif de réglage (5) pour régler le dispositif de restriction (2) en réaction aux informations obtenues.

60. Appareil selon la revendication 37, dans lequel ladite télécommande est capable d'obtenir des informations concernant le passage de la nourriture dans l'estomac et l'oesophage et de commander le dispositif de réglage pour régler le dispositif de restriction en réaction aux informations obtenues.

61. Appareil selon l'une quelconque des revendications 1 ou 8, comprenant en outre une source d'énergie implantable pour faire marcher le dispositif de mise en fonctionnement et/ou pour fournir de l'énergie à d'autres composants énergivores de l'appareil, dans lequel l'énergie provenant de la source d'énergie peut être libérée depuis l'extérieur du corps du patient.

62. Appareil selon l'une quelconque des revendications 1 ou 8, comprenant en outre un dispositif de transmission d'énergie pour une transmission d'énergie sans fil.

63. Appareil selon les revendications 61 et 62, dans lequel le dispositif de transmission d'énergie transmet l'énergie sous une première forme et qui comprend en outre un dispositif de transformation d'énergie implantable dans le patient pour transformer l'énergie sous une première forme en énergie sous une seconde forme qui alimentera la source d'énergie et/ou d'autres parties implantables énergivores de l'appareil.

64. Appareil selon la revendication 63, dans lequel la seconde forme d'énergie est différente de la première forme d'énergie.

65. Appareil selon la revendication 63, dans lequel le dispositif de transmission d'énergie fonctionne différemment du dispositif de transformation d'énergie.

66. Appareil selon la revendication 62, comprenant en outre un moteur électrique ou une pompe implantable pour faire fonctionner le dispositif de réglage, dans lequel le dispositif de transmission d'énergie est apte à transmettre de l'énergie sans fil sous forme d'un champ magnétique ou d'ondes ou de champ électromagnétiques pour une alimentation électrique directe du moteur ou de la pompe lorsque l'énergie sans fil est en cours de transmission.

67. Appareil selon les revendications 40 et 62, dans lequel le dispositif de transmission d'énergie transmet l'énergie par au moins un signal séparé du signal de commande.

68. Appareil selon la revendication 63, comprenant en outre un stabilisateur implantable pour stabiliser l'énergie sous sa première ou sa seconde forme.

69. Appareil selon la revendication 68, dans lequel l'énergie sous la seconde forme comprend le courant électrique et le stabilisateur comprend au moins un condensateur.

70. Appareil selon l'une quelconque des revendications 61 et 63 à 65, dans lequel la source d'énergie comprend une pile, un accumulateur, un condensateur ou une combinaison de ceux-ci.

71. Appareil selon l'une quelconque des revendications 1 ou 8, comprenant en outre un dispositif de commande apte à produire de l'énergie sans fil pour faire marcher directement le dispositif de mise en fonctionnement et/ou pour fournir de l'énergie à d'autres composants énergivores de l'appareil.

72. Appareil selon l'une quelconque des revendications 1 ou 8, comprenant en outre un dispositif de transformation d'énergie implantable pour transformer l'énergie sans fil directement ou indirectement en une énergie différente de l'énergie sans fil pour faire fonctionner le dispositif de restriction.

73. Appareil selon la revendication 71 ou 72, dans lequel l'énergie sans fil comprend un signal ondulatoire incluant un signal d'onde acoustique, un signal d'ultrasons, un signal d'onde électromagnétique, un signal de lumière infrarouge, un signal de lumière visible, un signal de lumière ultraviolette, un signal de lumière laser, un signal de micro-ondes ou un signal d'ondes radio.

74. Appareil selon l'une quelconque des revendications 1 ou 8, comprenant en outre un moyen de transfert d'énergie (22, 326, 332-344) pour un transfert sans fil d'énergie de l'extérieur du corps du patient vers le dispositif de mise en fonctionnement ou de réglage et/ou d'autres composants implantables énergivores de l'appareil.

75. Appareil selon la revendication 71, dans lequel le dispositif de commande est apte à produire de l'énergie sans fil sous la forme d'un train d'impulsions énergétiques.

76. Appareil selon la revendication 45 ou 74, dans lequel le moyen de transfert d'énergie est apte à transférer par intervalles l'énergie sous la forme d'un train d'impulsions énergétiques pour une utilisation directe en liaison avec la fourniture d'énergie aux composants énergivores de l'appareil.

77. Appareil selon les revendications 71 et 63, dans lequel le dispositif de commande est apte à commander le dispositif de transformation d'énergie pour produire de l'énergie sous la seconde forme d'un train d'impulsions énergétiques pour une utilisation directe en liaison avec le fonctionnement du dispositif de réglage.

78. Appareil selon la revendication 76 ou 77, dans lequel le dispositif de transfert d'énergie est apte de l'énergie électrique et qui comprend en outre un condensateur implantable pour produire le train d'impulsions énergétiques.

79. Appareil selon les revendications 78 ou 69, 70, dans lequel le condensateur a une capacité inférieure à 0,1 µF.

80. Appareil selon la revendication 74, comprenant en outre un moteur électrique (22) ou une pompe implantable pour faire fonctionner le dispositif de réglage (12 ; 52 ; 66 ; 90, 92 ; 104 ; 110), dans lequel le moyen de transfert d'énergie est apte à faire marcher directement le moteur électrique ou la pompe avec l'énergie transférée.

81. Appareil selon la revendication 66 ou 80, dans lequel la pompe n'est pas une pompe du type à plongeur.

82. Appareil selon l'une quelconque des revendications 38 à 81, comprenant en outre une télécommande sans fil (44, 126, 132-144) pour commander de façon non effractive le dispositif de mise en fonctionnement (6, 8 ; 10 ; 12 ; 10, 14 ; 76-86).

83. Appareil selon l'une quelconque des revendications précédentes, dans lequel le dispositif de mise en fonctionnement marche à l'électricité.

84. Appareil selon l'une quelconque des revendications 1, 8 ou 66, dans lequel le dispositif de mise en fonctionnement ne peut pas marcher avec une énergie magnétique permanente statique.

85. Appareil selon l'une quelconque des revendications 1 ou 8, dans lequel le dispositif de mise en fonctionnement est apte à faire fonctionner de façon non effractive le dispositif de réglage.

86. Appareil selon l'une quelconque des revendications 1 ou 8, dans lequel le moyen de restriction peut fonctionner pour régler de façon continue la restriction du passage de la nourriture.

87. Appareil selon la revendication 1 ou 8, dans lequel le dispositif de mise en fonctionnement comprend un dispositif de commande hydraulique qui utilise du fluide hydraulique dont la viscosité varie quand celui-ci est exposé à des formes d'énergie différentes de l'énergie thermique.

88. Appareil selon la revendication 83, dans lequel la viscosité du fluide hydraulique varie quand celui-ci est exposé à l'énergie électrique.

89. Appareil selon la revendication 1 ou 8, comprenant en outre un dispositif de réglage pour régler le dispositif de restriction afin de modifier la restriction du passage d'entrée de la nourriture, dans lequel le dispositif de réglage est apte à régler mécaniquement le dispositif de restriction ou apte à régler hydrauliquement le dispositif de restriction à l'aide d'un moyen hydraulique qui ne comporte pas de fluide hydraulique du genre de ceux dont la viscosité augmente sensiblement quand ils sont exposés à la chaleur ou à un champ magnétique.

90. Appareil selon la revendication 1 ou 8, comprenant en outre un dispositif de commande pour commander le dispositif de restriction.

91. Appareil selon la revendication 90, dans lequel le dispositif de commande comprend une unité de commande interne implantable dans le patient pour commander le dispositif de restriction.

92. Appareil selon la revendication 91, dans lequel l'unité de commande interne est programmable.

93. Appareil selon la revendication 92, dans lequel le dispositif de commande comprend une unité de commande externe à l'extérieur du corps du patient, l'unité de commande interne implantable étant programmable par l'unité de commande externe.

94. Appareil selon la revendication 90, dans lequel le dispositif de commande comprend une unité de commande externe à l'extérieur du corps du patient pour commander sans fil le dispositif de restriction.

95. Appareil selon la revendication 94, dans lequel l'unité de commande externe est programmable.

96. Appareil selon l'une quelconque des revendications précédentes, comprenant en outre au moins une sonde implantable pour capter au moins un paramètre physique du patient.

97. Appareil selon la revendication 96, dans lequel la sonde est apte à capter, directement ou indirectement, comme paramètre physique la position horizontale du patient.

98. Appareil selon la revendication 96, dans lequel la sonde comprend un détecteur de pression pour capter, directement ou indirectement, comme paramètre physique la pression contre le dispositif de restriction ou une partie du corps humain.

99. Appareil selon la revendication 98, dans lequel le moyen de restriction est apte à agrandir le passage de la nourriture dans l'estomac ou l'oesophage en réaction à la détection d'une pression déterminée par le détecteur de pression.

100. Appareil selon l'une quelconque des revendications 96 à 99, comprenant en outre un dispositif de commande pour commander le dispositif de restriction en réaction à des signaux provenant de la sonde.

101. Appareil selon la revendication 100, dans lequel le moyen de commande comprend une unité de commande interne implantable dans le patient et commandant directement le dispositif de restriction en réaction à des signaux provenant de la sonde.

102. Appareil selon la revendication 101, dans lequel le moyen de commande comprend une unité de commande externe à l'extérieur du corps du patient pour commander le dispositif de restriction en réaction à des signaux provenant de la sonde.

103. Appareil selon la revendication 101, dans lequel le moyen de commande comprend une unité de commande externe à l'extérieur du corps du patient pour commander manuellement le dispositif de restriction en réaction à des informations provenant de la sonde.

104. Appareil selon la revendication 90, comprenant en outre une source d'énergie implantable, dans lequel on peut faire fonctionner le dispositif de commande de l'extérieur du corps du patient pour commander à la source d'énergie de libérer de l'énergie à utiliser en liaison avec le fonctionnement de la prothèse, quand une prothèse est implantée.

105. Appareil selon la revendication 104, dans lequel la source d'énergie est censée être extérieure au corps du patient et le dispositif de commande est apte à commander à la source d'énergie externe de libérer de l'énergie sans fil à utiliser en liaison avec le fonctionnement de la prothèse.

106. Appareil selon la revendication 104, dans lequel le dispositif de commande commande à la source d'énergie de libérer de l'énergie magnétique, de l'énergie non magnétique, de l'énergie électromagnétique, de l'énergie non électromagnétique, de l'énergie cinétique, de l'énergie non cinétique, de l'énergie acoustique, de l'énergie non acoustique, de l'énergie thermique ou de l'énergie non thermique.

107. Appareil selon la revendication 104, dans lequel le dispositif de commande commande à la source d'énergie de libérer de l'énergie pendant une période déterminée.

108. Appareil selon les revendications 104 ou 104 et 75, dans lequel le dispositif de commande commande à la source d'énergie de libérer de l'énergie en un nombre déterminé d'impulsions énergétiques.

109. Appareil selon la revendication 104, dans lequel le dispositif de commande est apte à commander à la source d'énergie de libérer de l'énergie de manière non effractive.

110. Appareil selon l'une quelconque des revendications 1 ou 8, comprenant en outre des composants électriques implantables y compris au moins un dispositif de protection de niveau de tension.

111. Appareil selon l'une quelconque des revendications 1 ou 8, comprenant en outre des composants électriques implantables y compris un seul dispositif de protection de niveau de tension.

112. Appareil selon la revendication 110 ou 111, dans lequel les composants électriques sont dépourvus de tout détecteur de courant et/ou détecteur de niveau de charge.

113. Appareil selon l'une quelconque des revendications 61, 78, 63, 69 ou 70 et 110 à 112, comprenant en outre un condensateur ou un accumulateur implantable, dans lequel la charge ou décharge du condensateur ou de l'accumulateur est commandée au moyen du dispositif de protection de niveau de tension.

114. Appareil selon la revendication 40, 46 ou 67, dans lequel le signal de commande comprend un signal ondulatoire incluant un signal d'onde acoustique, un signal d'ultrasons, un signal d'onde électromagnétique, un signal de lumière infrarouge, un signal de lumière visible, un signal de lumière ultraviolette, un signal de lumière laser, un signal de micro-ondes ou un signal d'ondes radio.

115. Appareil selon l'une quelconque des revendications précédentes, comprenant en outre un interrupteur implantable pour commuter directement ou indirectement le fonctionnement du dispositif de restriction.

116. Appareil selon les revendications 115 et 62, comprenant en outre une source d'énergie implantable dans le patient pour fournir de l'énergie pour le fonctionnement du dispositif de restriction, dans lequel l'interrupteur est actionné par l'énergie fournie par le dispositif de transmission d'énergie pour commuter d'un mode arrêt, dans lequel la source d'énergie n'est pas en service, à un mode marche, dans lequel la source d'énergie fournit de l'énergie pour le fonctionnement du dispositif de restriction.

117. Appareil selon les revendications 115 et 62, comprenant en outre une source d'énergie implantable dans le patient, pour fournir de l'énergie pour le fonctionnement du dispositif de restriction, et une télécommande pour commander l'alimentation en énergie de la source d'énergie implantable, dans lequel l'interrupteur est actionné par l'énergie fournie par le dispositif de transmission d'énergie pour commuter d'un mode arrêt, dans lequel la télécommande est empêchée de commander la source d'énergie et la source d'énergie n'est pas en service, à un mode Attente, dans lequel la télécommande a la possibilité de commander à la source d'énergie de fournir de l'énergie pour le fonctionnement du dispositif de restriction.

118. Appareil selon les revendications 115 et 63, comprenant en outre une source d'énergie implantable dans le patient pour fournir de l'énergie pour le fonctionnement du dispositif de restriction, dans lequel l'interrupteur est actionné par l'énergie fournie par le dispositif de transformation d'énergie pour commuter d'un mode arrêt, dans lequel la source d'énergie n'est pas en service, à un mode marche, dans lequel la source d'énergie fournit de l'énergie pour le fonctionnement du dispositif de restriction.

119. Appareil selon les revendications 115 et 63, comprenant en outre une source d'énergie implantable dans le patient, pour fournir de l'énergie pour le fonctionnement du dispositif de restriction, et une télécommande pour commander l'alimentation en énergie de la source d'énergie implantable, dans lequel l'interrupteur est actionné par l'énergie fournie par le dispositif de transformation d'énergie pour commuter d'un mode arrêt, dans lequel la télécommande est empêchée de commander la source d'énergie et la source d'énergie n'est pas en service, à un mode Attente, dans lequel la télécommande a la possibilité de commander à la source d'énergie de fournir de l'énergie pour le fonctionnement du dispositif de restriction.

120. Appareil selon l'une quelconque des revendications 1 ou 8, dans lequel la prothèse peut fonctionner pour remplir une fonction réversible.

121. Appareil selon la revendication 42 ou 120, comprenant en outre un dispositif d'inversion implantable dans le patient pour inverser la fonction remplie par la prothèse.

122. Appareil selon la revendication 121, dans lequel le dispositif de commande commande au dispositif d'inversion d'inverser la fonction remplie par la prothèse.

123. Appareil selon la revendication 121, dans lequel le dispositif d'inversion comprend un moyen hydraulique incluant une valve pour modifier le sens d'écoulement d'un fluide s'écoulant dans le moyen hydraulique.

124. Appareil selon la revendication 121, dans lequel le dispositif d'inversion comprend un moyen d'inversion mécanique.

125. Appareil selon la revendication 121, dans lequel le dispositif d'inversion comprend un commutateur.

126. Appareil selon la revendication 125, dans lequel le commutateur du dispositif d'inversion peut être actionné par l'énergie libérée.

127. Appareil selon la revendication 126, dans lequel le dispositif de commande commande le fonctionnement du commutateur du dispositif d'inversion en modifiant la polarité de l'énergie libérée alimentant le commutateur.

128. Appareil selon la revendication 121, dans lequel le dispositif de mise en fonctionnement comprend un moteur électrique et le dispositif d'inversion inverse le moteur.

129. Appareil selon l'une quelconque des revendications précédentes, dans lequel le dispositif de restriction est noyé dans une matière molle ou ayant la consistance d'un gel.

130. Appareil selon la revendication 129, dans lequel le dispositif de restriction est noyé dans une matière silicone ayant une dureté inférieure à 20 Shore.

131. Appareil selon la revendication 63, dans lequel le moyen ou dispositif de transformation d'énergie est conçu pour être implanté de manière sous-cutanée ou dans l'abdomen, le thorax ou la région céphalique du patient.

132. Appareil selon l'une quelconque des revendications précédentes, dans lequel le dispositif de réglage est apte à régler le dispositif de restriction de telle sorte que celui-ci assure une contraction prédéterminée du passage de la nourriture qui soit satisfaisante pour le patient.

133. Appareil selon l'une quelconque des revendications 1 ou 8, dans lequel le dispositif de réglage est apte à régler le dispositif de restriction d'une manière qui ne mette en oeuvre ni flux magnétique, ni effet thermique ni modification de viscosité.

134. Appareil selon la revendication 1, dans lequel le moyen de réglage comprend une cavité expansible dans l'élément de restriction, le passage de la nourriture est limité quand la cavité se dilate et agrandi quand la cavité se contracte et le moyen hydraulique de mise en fonctionnement comprend un orifice d'injection implantable de manière sous-cutanée dans le patient pour, par voie transcutanée, ajouter du fluide dans la cavité ou en retirer de celle-ci.

135. Appareil selon la revendication 1 ou 8, dans lequel le moyen de réglage comprend un moyen hydraulique de mise en fonctionnement et ledit moyen hydraulique de mise en fonctionnement comprend un orifice d'injection implantable de manière sous-cutanée dans le patient pour, par voie transcutanée, ajouter du fluide dans la cavité ou en retirer de celle-ci.

136. Appareil selon la revendication 135, dans lequel l'orifice d'injection n'est utilisé qu'à des fins d'étalonnage et le dispositif de réglage est en outre capable d'augmenter l'ouverture de limitation de la nourriture quand la nourriture doit passer lorsque le patient est en train de manger et avale de la nourriture.

137. Appareil selon les revendications 136 et 8, dans lequel le servomécanisme fonctionne hydrauliquement et dans lequel l'orifice d'injection n'est utilisé qu'à des fins d'étalonnage du servomécanisme.

138. Appareil selon la revendication 2, dans lequel les première et seconde parties (66) de cloison du réservoir sont conçues pour pouvoir être déplacées l'une par rapport à l'autre par manipulation manuelle de celles-ci.

139. Appareil selon l'une quelconque des revendications précédentes, comprenant en outre au moins un dispositif de maintien implantable dans le patient pour maintenir l'oesophage ou l'estomac dans une position où se situent les piliers tendieux droit et gauche, afin d'empêcher la région du cardia de se déplacer vers le haut au travers du muscle diaphragmatique.

140. Appareil selon la revendication 139, dans lequel le dispositif de maintien est placé en contact mécanique avec l'appareil de limitation de la nourriture.

141. Appareil selon la revendication 1 ou 8, dans lequel on peut faire fonctionner le moyen de restriction pour ouvrir et fermer le passage de la nourriture.

142. Appareil selon la revendication 141, dans lequel on peut faire fonctionner le moyen de restriction pour ouvrir le passage de la nourriture quand la nourriture doit passer et que le patient est en train d'avaler et autrement fermer le passage de la nourriture afin d'empêcher un reflux d'acide depuis l'estomac.

143. Appareil selon la revendication 1 ou 8, dans lequel on peut faire fonctionner le moyen de restriction pour régler de façon progressive la limitation du passage de nourriture.

144. Appareil selon la revendication 1 ou 8, dans lequel le moyen de réglage est motorisé.

145. Appareil selon la revendication 1 ou 8, dans lequel le dispositif de mise en fonctionnement est motorisé.

146. Appareil selon la revendication 1 ou 8, dans lequel on peut faire fonctionner le moyen de réglage de manière non effractive.

147. Appareil selon la revendication 1 ou 8, dans lequel on peut faire fonctionner le dispositif de mise en fonctionnement de manière non effractive.
